# EUROPÄISCHE PATENTSCHRIFT

(11) **EP 4 438 063 B1**
(45) Veröffentlichungstag und Bekanntmachung des Hinweises auf die Patenterteilung: **28.05.2025**
(21) Anmeldenummer: 23166186.9
(22) Anmeldetag: 31.03.2023
(51) Int. Cl.: A61L 2/00, A61C 19/00, A61L 2/26, A61B 90/70, A61M 39/16, A61C 1/10, A61C 1/12

(54) **ADAPTER ZUM VERBINDEN EINES HOHLKÖRPEROBJEKTS MIT EINEM GERÄT ZUR INNENDURCHSPÜLUNG VON HOHLKÖRPEROBJEKTEN**
ADAPTER FOR CONNECTING A HOLLOW-BODY OBJECT TO A DEVICE FOR WASHING THROUGH THE INSIDE OF HOLLOW-BODY OBJECTS
ADAPTATEUR POUR RELIER UN OBJET À CORPS CREUX À UN APPAREIL POUR LE RINÇAGE INTÉRIEUR D'OBJETS À CORPS CREUX

(43) Veröffentlichungstag der Anmeldung: 02.10.2024
(73) Patentinhaber: MELAG Medizintechnik GmbH & Co. KG, 10829 Berlin (DE)
(72) Erfinder: WOHLFAHRT, Viktor, 12053 Berlin (DE); SANTNER, Patrick, 14641 Nauen (DE)
(74) Vertreter: Maikowski & Ninnemann Patentanwälte Partnerschaft mbB

(56) Entgegenhaltungen:
- EP-A1- 2 946 749
- EP-A1- 3 248 571
- DE-A1- 102019 209 045
- DE-B3- 102008 023 458

## Beschreibung

Die vorliegende Erfindung betrifft einen Adapter zum Verbinden eines Hohlkörperobjekts mit einem Gerät zur Innendurchspülung von Hohlkörperobjekten gemäß dem Oberbegriff des Anspruchs 1, eine Adapteranordnung mit einem solchen Adapter gemäß dem Oberbegriff des Anspruchs 14 und ein Verfahren zum Verbinden eines Hohlkörperobjekts mit einem Gerät zur Innendurchspülung von Hohlkörperobjekten und zum Lösen des Hohlkörperobjekts von dem Gerät gemäß dem Oberbegriff des Anspruchs 15.

Insbesondere in der Zahnmedizin werden Instrumente eingesetzt, die schlecht zugängliche Hohlräume aufweisen. Beispiele für derartige Instrumente sind Hand- und Winkelstücke oder Turbinen. Weil sich Mikroorganismen insbesondere an solchen schlecht zugänglichen Hohlräumen wie etwa Spalten, Riefen oder Poren ansammeln, an denen sie auch ausreichend Nährstoffe vorfinden, stellen derartige Hohlräume eine besondere Herausforderung hinsichtlich der Reinigung, Desinfektion, Sterilisation und Trocknung dar.

Reinigungs- und Desinfektionsgeräte führen typischerweise eine Innendurchspülung von ärztlichen und zahnärztlichen Instrumenten durch, um Mikroorganismen an den schwer zugänglichen Hohlräumen abzutöten und Verschmutzungen im Inneren der Instrumente zu beseitigen. Zu diesem Zweck sind regelmäßig Adapter vorgesehen, mit denen die entsprechenden Instrumente mit den Reinigungs- und Desinfektionsgeräten verbunden werden.

Übertragungsinstrumente wie etwa Hand- und Winkelstücke weisen zudem ein im Innenraum der Übertragungsinstrumente befindlichen Antrieb auf, der gesondert gepflegt werden muss. So wird von den Herstellern von Übertragungsinstrumenten eine Pflege mit einem speziell dafür vorgesehenem Dentalöl typischerweise mindestens einmal am Tag nach der Reinigung und Desinfektion (jedoch vor einer Sterilisation) empfohlen. Dieses Dentalöl wird üblicherweise nur in einen Antriebskanal des Übertragungsinstruments eingebracht, da sich nur im Antriebskanal bewegliche Teile, die mittels des Dentalöls gepflegt werden müssen, befinden. Demgegenüber werden sogenannte Spraykanäle in Übertragungsinstrumenten lediglich gereinigt und desinfiziert, nicht jedoch mit Dentalöl versorgt. Durch die Trennung von Spray- und Antriebskanälen ist eine isolierte Versorgung der beweglichen Antriebselemente mit Dentalöl möglich.

Die Adapter, mit denen die Übertragungsinstrumente an ein Reinigungs- und Desinfektionsgerät angeschlossen werden, weisen typischerweise ebenfalls unterschiedliche Kanäle zur getrennten Kontaktierung der Spray- und Antriebskanäle in den Übertragungsinstrumenten auf.

Die zur Verbindung der Instrumente mit einem Reinigungs- und Desinfektionsgerät eingesetzten Adapter müssen eine ausreichend große Haltekraft bereitstellen, um dem vom Reinigungs- und Desinfektionsgerät aufgebrachten Spüldruck zuverlässig standzuhalten. Darüber hinaus sollen sie ein schnelles und anwenderfreundliches Verbinden und Lösen des Instruments mit/von dem Reinigungs- und Desinfektionsgerät ermöglichen. Ferner soll eine verlässliche Versorgung der im Instrument befindlichen Kanäle mit dem dafür vorgesehenen Medium (insbesondere nur Spülflüssigkeit bzw. Spülflüssigkeit und dentale Öl) und gegebenenfalls eine Abdichtung gegenüber der Umgebung sichergestellt werden. Schließlich sollen Spülschatten minimiert werden, damit möglichst keine ungereinigten Bereiche der Übertragungsinstrumente bzw. anderen Instrumente resultieren.

Die Medienkanäle der Adapter werden auch dafür eingesetzt, um Trocknungsluft in zuvor gereinigte und desinfizierte Gegenstände zu leiten. Diese Trocknungsluft dient der Beschleunigung der Trocknung bzw. der Reduzierung der Restfeuchtigkeit. Dabei wird grundsätzlich zwischen der Innentrocknung und der Außentrocknung der zu trocknenden Gegenstände unterschieden.

Bei der Innentrocknung wird die Trocknungsluft durch ein Innenlumen eines zu trocknenden Instrumentes geleitet. Die Trocknung wird durch die aus der thermischen Desinfektion der Instrumente resultierenden Eigenwärme der Instrumente erreicht und durch die durch die Instrumente durchgeleitete Trocknungsluft beschleunigt.

Die DE 296 24 425 U1 beschreibt eine Spülvorrichtung zur Innendurchspülung und Halterung zahnärztlicher Hand- und Winkelstücke, die in einem Instrumenteneinsatz, einem Einsatzwagen oder dergleichen angeordnet ist. Der Instrumenteneinsatz ist in der Spülkammer einer Reinigungsvorrichtung wie etwa einer programmgesteuerten Spülmaschine separat einsetzbar und wird mit vorgefilterter Spül- und Reinigungsflüssigkeit versorgt. Die Spülvorrichtung im Instrumenteneinsatz ist als mehrteiliger Halter mit einem herausnehmbaren Mikrofilter für die Spül- und Reinigungsflüssigkeit ausgebildet.

Die DE 10 2014 111 237 A1 betrifft eine Sprühvorrichtung zur Innendurchspülung von dentalem Spülgut, insbesondere von Turbinen sowie von Hand- und Winkelstücken. Die Sprühvorrichtung weist einen Kupplungsabschnitt auf, der für einen Anschluss des Spülguts an einen Anschlussstutzen einer Rohrleitung sorgt. Ferner ist eine Düse vorgesehen, die einen konisch zulaufenden Düsenhals aufweist. Außerdem weist die Sprühvorrichtung eine Hülse auf, die mit einem längsgeschlitzt ausgebildeten Endabschnitt den Düsenhals aufnimmt. Die Hülse ist dabei derart an der Düse angeordnet, dass sie in Düsenhöhenrichtung relativ verfahrbar zur Düse ist.

Die EP 2 946 749 A1 beschreibt ebenfalls eine Vorrichtung zur Innendurchspülung von dentalem Spülgut wie Turbinen sowie Hand- und Winkelstücken. Diese Vorrichtung weist ebenfalls eine Kupplungseinrichtung zum Anschluss des Spülguts an einen mit einer Anschlusseinrichtung einer Rohrleitung verbundenen Anschlussstutzen auf. Die Kupplungseinrichtung weist eine Klemmeinrichtung zur Lagefixierung eines an die Kupplungseinrichtung angeschlossenen Spülguts auf. Dabei ist die Kupplungseinrichtung zur Betätigung der Klemmeinrichtung relativ verfahrbar zum Anschlussstutzen ausgebildet.

Die DE 10 2021 119 351 A1 beschreibt eine Adaptionsvorrichtung zum Aufnehmen von medizinischem Reinigungsgut. Diese Adaptionsvorrichtung weist eine Hülseneinheit auf, in der ein Hohlraum ausgeformt ist. Die Hülseneinheit weist dabei einen Verbindungsabschnitt zum fluidmechanischen Verbinden des Hohlraums mit einer Zufuhreinrichtung eines Spülkorbs eines Reinigungsgeräts und eine dem Verbindungsabschnitt gegenüberliegende Hülsenöffnung auf. Ferner weist die Adaptionsvorrichtung eine Haltereinrichtung auf, die dazu dient, das medizinische Reinigungsgut in einer mit der Trägereinrichtung gekoppelten Position zu halten. Zudem umfasst die Adaptionsvorrichtung ein elastisches Mittel, das im Hohlraum der Hülseneinheit angeordnet ist und dafür dient, die Trägereinrichtung in Richtung der Hülsenöffnung vorzuspannen.

Die DE 10 2016 102 907 A1 beschreibt eine Haltevorrichtung für einen Kopf eines medizinischen Instruments in einem Reinigungs- und Desinfektionsgerät. Die Haltevorrichtung weist eine Auslassdüse und einen im Bereich der Auslassdüse schwenkbar gehaltenen Klemmbügel auf. Der Klemmbügel dient dazu, mit einem Drehmomentschlüssel, in den der Kopf eingesetzt ist, zusammenzuwirken, um den Kopf in Bezug auf die Auslassdüse zu fixieren.

Die EP 3 248 571 A1 beschreibt einen Anschlussadapter für den strömungstechnischen Anschluss eines dentalen Spülguts an einen Anschlussstutzen einer Rohrleitung. Der Anschlussadapter weist einen Grundkörper mit einer Spülkammer zur Aufnahme des Spülguts auf. Eine in dem Grundkörper ausgebildete Beschickungsöffnung kann durch ein beweglich am Grundkörper angeordnetes Verschlussteil verschlossen werden.

Die EP 0 937 441 A1 beschreibt eine Vorrichtung zum Reinigen zahnärztlicher Gegenstände. Diese Vorrichtung umfasst eine Kassette zur Aufnahme der zu reinigenden Gegenstände sowie eine druckdicht verschließbare Kammer, in die die Kassette eingelegt wird. Zur Halterung der zu reinigenden Gegenstände sind in der Kassette Adapter vorgesehen.

Die aus dem Stand der Technik bekannten Lösungen weisen verschiedene Nachteile auf. Teilweise realisieren die Adapter lediglich eine kraftschlüssige Verbindung mit dem zu reinigenden Spülgut. Wenn der Spüldruck des Reinigungs- und Desinfektionsgeräts einer Federkraft des Adapters entgegenwirkt, muss die Federkraft ausreichend groß sein, um auch bei hohem Spüldruck eine sichere Verbindung des Spülguts am Adapter zu gewährleisten. Dann muss diese hohe Federkraft allerdings auch überwunden werden, um das Spülgut am Adapter zu befestigen oder es vom Adapter zu lösen. Hohe Spüldrücke werden angestrebt, um erhöhten Anforderungen an die Reinigung von Instrumenten gerecht zu werden und um komplexere Instrumente aufzubereiten. Denn grundsätzlich können komplexe Innengeometrien umso besser durchspült werden, je höher der Spüldruck ist

Je nach Geometrie einer in dem Spülgut ausgebildeten Nut, die dazu vorgesehen ist, dass ein Hebel des Adapters in sie eingreift, kann auch eine formschlüssige Verbindung realisiert werden. Dann wirkt der Spüldruck der entsprechenden Federkraft nicht entgegen. Allerdings müssen die Geometrie des Adapters und des an den Adapter anzuschließenden Instruments dann genau aufeinander abgestimmt sein.

Bei zahlreichen der aus dem Stand der Technik bekannten Lösungen decken die Adapter zudem einen Teil der zu reinigenden Flächen des an den Adapter anzuschließenden Instruments ab, sodass ein Spülschatten entsteht.

Mitunter sind auch zusätzliche Werkzeuge wie etwa in Drehmomentschlüssel erforderlich, um eine Verbindung zwischen dem Adapter und dem zu reinigenden Instrument realisieren zu können.

Wieder andere aus dem Stand der Technik bekannte Lösungen erfordern einen großen Bauraum und können aufgrund der vorgesehenen Konstruktion keinen sicheren Halt der zu reinigenden Instrumente am Adapter gewährleisten.

Der vorliegenden Erfindung liegt die Aufgabe zugrunde, einen Adapter zum Verbinden eines Hohlkörperobjekts (beispielsweise eines medizinischen Instruments) mit einem Gerät zur Innendurchspülung des Hohlkörperobjekts bereitzustellen, wobei der Adapter die aus dem Stand der Technik bekannten Nachteile überwindet. Insbesondere soll der Adapter universell eingesetzt werden können, eine Reinigung des Hohlkörperobjekts ohne Spülschatten ermöglichen und für einen Benutzer einfach handhabbar sein.

Diese Aufgabe wird durch einen Adapter zum Verbinden eines Hohlkörperobjekts mit einem Gerät zur Innendurchspülung von Hohlkörperobjekten, der die Merkmale des Anspruchs 1 aufweist, gelöst. Ein solcher Adapter weist ein Innenteil und ein das Innenteil zumindest abschnittsweise umgebendes Außenteil auf. Das Innenteil und das Außenteil erstrecken sich dabei entlang einer Längserstreckungsachse und sind relativ zueinander beweglich. Das Außenteil weist eine Außenteilwandung mit mindestens einer Rasteinrichtung auf.

Erfindungsgemäß ist vorgesehen, dass die Rasteinrichtung einen Durchbruch in der Außenteilwandung, einen in dem Durchbruch beweglich gelagerten Körper und eine Bewegungsbegrenzungseinrichtung für den Körper umfasst. Der Durchbruch weist dabei eine erste Öffnung und eine zweite Öffnung auf. Die erste Öffnung ist in einer Innenseite der Außenteilwandung realisiert, die zum Innenteil des Adapters hin orientiert ist. Die zweite Öffnung ist in einer Außenseite der Außenteilwandung realisiert, die vom Innenteil weg orientiert ist. Von der ersten Öffnung zur zweiten Öffnung verläuft eine Radialrichtung des Adapters.

Die Bewegungsbegrenzungseinrichtung begrenzt eine in der Radialrichtung zur zweiten Öffnung hin verlaufende Bewegung des Körpers. Dabei verhindert die Bewegungsbegrenzungseinrichtung, dass der Körper vollständig durch die zweite Öffnung nach außen durchtreten kann. Mit anderen Worten ausgedrückt, kann der Körper wegen der Bewegungsbegrenzungseinrichtung den Durchbruch durch die zweite Öffnung nicht vollständig verlassen, kann sich im Durchbruch aber grundsätzlich in der Radialrichtung bewegen.

Die erste Öffnung weist einen Durchmesser auf, der größer ist als ein Querschnittsdurchmesser des Körpers. Mithin ist es für den Körper grundsätzlich möglich, durch die erste Öffnung nach außen zu treten und den Durchbruch so zumindest teilweise zu verlassen.

Die Rasteinrichtung kann zwischen einer Freigabeposition und einer Rastposition verstellt werden. Dies erfolgt durch eine Relativbewegung zwischen dem Außenteil und dem Innenteil des Adapters.

In der Rastposition ist der Körper in dem Durchbruch radial weiter außen angeordnet als in der Freigabeposition. Schließlich ragt eine Außenseite der Rasteinrichtung in der Freigabeposition nicht über eine Außenumhüllende der Außenteilwandung hinaus. Demgegenüber ragt die Außenseite der Rasteinrichtung in der Rastposition über die Außenumhüllende der Außenteilwandung hinaus. Durch dieses Hinausragen über die Außenumhüllende der Außenteilwandung kann die Rasteinrichtung in der Rastposition eine Rastfunktion ausüben.

Mit solch einem Adapter kann eine formschlüssige Verbindung zu einem Hohlkörperobjekt mit einer radial innenliegenden Nut (also auf einer Innenseite des Hohlkörperobjekts, die nach dem Verbinden des Hohlkörperobjekts mit dem Adapter einer Außenseite des Adapters zugewandt ist) realisiert werden. Dabei ist die Außengeometrie des Adapter-Außenteils unabhängig von der Rastfunktion, die durch die Rasteinrichtung bereitgestellt wird. Mithin lassen sich Adapter mit unterschiedlichsten Außengeometrien realisieren, sodass eine passgenaue Adaptierung unterschiedlichster Hohlkörperobjekte möglich wird.

Der Adapter benötigt nur einen geringen Bauraum in einem Reinigungs- und Desinfektionsgerät. Dies ist vorteilhaft in Bezug auf die Bestückungsmöglichkeiten auf einem Trägersystem des Reinigungs- und Desinfektionsgeräts. Es können beim Einsatz des vorliegend beanspruchten Adapters mithin auf engem Raum zahlreiche unterschiedliche Hohlkörperobjekte gleichzeitig gereinigt werden, die jeweils an einen eigenen Adapter angekoppelt werden.

Die zum Lösen eines an den Adapter gekoppelten Hohlkörperobjekts erforderliche Lösekraft ist unabhängig von der Haltekraft. Dadurch ergibt sich eine einfache Bedienung für einen Benutzer. Zudem bleibt ein Hohlkörperobjekt sicher mit dem Adapter verbunden, auch wenn ein hoher Spüldruck von einem Reinigungs- und Desinfektionsgerät aufgebracht wird und bei der Innendurchspülung des Hohlkörperobjekts auf das Hohlkörperobjekt wirkt.

Eine unzureichende Verbindung zwischen dem zu reinigenden Hohlkörperobjekt und dem Adapter wird ebenso wie eine unzureichende Reinigung des Hohlkörperobjekts im Gegensatz zu den aus dem Stand der Technik bekannten universellen Adapterkonzepten sicher vermieden. Zudem bildet der Adapter keinen Spülschatten auf dem zu reinigenden Hohlkörperobjekt, sodass eine gleichmäßige und gründliche Reinigung des Hohlkörperobjekts möglich wird.

In dem Adapter können in beliebiger Weise Kanäle ausgebildet werden, sodass die unterschiedlichen Medienkanäle innerhalb des Hohlkörperobjekts getrennt voneinander gereinigt werden können. Mithin ist es möglich - wie dies bereits auch bei aus dem Stand der Technik bekannten Lösungen der Fall ist -, Spraykanäle und Antriebskanäle gesondert voneinander zu reinigen und wahlweise mit Dentalöl zu beschicken.

In einer Ausgestaltung handelt es sich bei dem Hohlkörperobjekt um ein medizinisches Instrument, insbesondere ein zahnmedizinisches Instrument. Insbesondere handelt es sich bei dem Hohlkörperobjekt um ein medizinisches oder zahnmedizinisches Übertragungsinstrument wie etwa ein Hand- oder Winkelstück. Eine Turbine ist ein weiteres als Hohlkörperobjekt geeignetes ärztliches oder zahnärztliches Instrument. All diese Instrumente weisen in ihrem Innern Medienkanäle wie Spray- und Antriebskanäle auf, die bei der Innendurchspülung der Instrumente gereinigt und optional gepflegt werden. Aufgrund dieser Kanäle können die Instrumente auch als Hohlkörperobjekte bezeichnet werden.

Der in dem Durchbruch der Rasteinrichtung beweglich gelagerte Körper kann grundsätzlich eine beliebige Ausgestaltung aufweisen. In einer Variante handelt es sich bei dem Körper um einen Zylinderstift mit abgerundeten Kuppen, um eine Formfeder oder um eine Kugel. Dabei ist die Ausgestaltung des Körpers als Kugel besonders für die Funktion der Rasteinrichtung geeignet. Wenn der beweglich gelagerte Körper als Kugel ausgestaltet ist, handelt es sich bei der Rasteinrichtung um eine Kugelrasteinrichtung. Die Kugel kann innerhalb dieser Kugelrasteinrichtung die Funktion eines Rastelements übernehmen oder aber ein von der Kugel separates Rastelement von der Freigabeposition in die Rastposition und von der Rastposition in die Freigabeposition überführen.

In einer Ausgestaltung ist die Bewegungsbegrenzungseinrichtung durch einen Sprengring ausgebildet. Dieser Sprengring ist in dem Durchbruch in der Radialrichtung außen um den Körper herum angeordnet. Wenn sich der Körper radial nach außen bewegt, wird der Sprengring nach außen gedrückt. Dann überragt die Rasteinrichtung eine Außenumhüllende der Außenteilwandung. Wird der Körper hingegen in Radialrichtung nach innen bewegt, nimmt der Sprengring wieder seine ursprüngliche Position ein, in der er nicht über die Außenumhüllende der Außenteilwandung hinausragt. Dann befindet sich die Rasteinrichtung in der Freigabeposition. Der als Bewegungsbegrenzungseinrichtung dienende Sprengring nimmt in dieser Ausgestaltung somit zugleich die Funktion eines Rastelements der Rasteinrichtung ein und tritt in der Rastposition in direkten Kontakt mit dem zu fixierenden Hohlkörperobjekt.

Um eine möglichst universelle Einsetzbarkeit des Adapters zu ermöglichen, weist der Sprengring in einer Variante keine besondere Außenkontur auf, sondern ist als klassischer Ring mit einem kreisförmigen Querschnitt ausgebildet.

In einer anderen Ausgestaltung weist der Sprengring einen von einer Kreisform abweichenden Querschnitt auf. Dann lassen sich spezifisch an zu kontaktierende Hohlkörperobjekte angepasste Ausgestaltungen der Außenseite des Sprengrings realisieren, die passgenau in eine innenliegende Nut in dem Hohlkörperobjekt eingefügt werden können. Dadurch lässt sich ein Hohlkörperobjekt besonders sicher kontaktieren. Allerdings lässt sich ein derart ausgestalteter Sprengring nicht so universell einsetzen wie ein Sprengring mit einer üblichen Außenkontur wie etwa einer genormten oder kreisförmigen Außenkontur.

In einer Ausgestaltung weist die Außenteilwandung im Bereich der Rasteinrichtung eine Wandstärke auf, die kleiner ist als eine Erstreckung des Körpers und des Sprengrings in der Radialrichtung. Dadurch wird es besonders einfach möglich, dass ein aus dem Körper und dem Sprengring gemeinsam gebildetes Rastelement der Rasteinrichtung über die Außenumhüllende der Außenteilwandung des Adapters hinausragen kann. Wenn der Körper hingegen radial nach innen teilweise durch die erste Öffnung aus dem Durchbruch hinausragt, ist es für den Sprengring problemlos möglich, dass sein Außenumfang mit der Außenteilwandung fluchtet, sodass die Rasteinrichtung nicht mehr über die Außenumhüllende der Außenteilwandung hinausragt. Mithin ist es bei dieser Variante problemlos möglich, dass durch eine Radialbewegung des Körpers eine Überführung der Rasteinrichtung von der Freigabeposition in die Rastposition und von der Rastposition in die Freigabeposition erfolgt.

In einer Ausgestaltung ist die Bewegungsbegrenzungseinrichtung durch einen Durchmesser der zweiten Öffnung ausgebildet, der kleiner als der Querschnittsdurchmesser des Körpers ist. In dieser Ausgestaltung wirkt der Körper direkt als Rastelement gegenüber dem zu fixierenden Hohlkörperobjekt. Das heißt, in dieser Variante ist kein zusätzlicher Sprengring erforderlich. Da der Durchmesser der zweiten Öffnung zudem kleiner als der Querschnittsdurchmesser des Körpers ist, kann der Körper nicht aus dem Durchbruch der Rasteinrichtung nach außen gelangen. Vielmehr kann er lediglich teilweise durch die Außenteilwandung hindurchtreten und somit über die Außenumhüllende der Außenteilwandung hinausragen. Bewegt sich der Körper in Radialrichtung jedoch nach innen (also zur ersten Öffnung der Rasteinrichtung hin oder zumindest teilweise durch die erste Öffnung hindurch), fluchtet eine Außenseite der Rasteinrichtung mit der Außenumhüllenden in der Außenteilwandung. Somit wird die Rasteinrichtung auch in dieser Ausgestaltung durch eine Radialbewegung des Körpers im Durchbruch von der Freigabeposition in die Rastposition und von der Rastposition in die Freigabeposition überführt.

In einer Ausgestaltung, insbesondere in einer Ausgestaltung, in der der Körper als Rastelement fungiert, weist die Außenteilwandung im Bereich der Rasteinrichtung eine Wandstärke auf, die kleiner ist als eine Erstreckung des Körpers in der Radialrichtung. Dann ragt der Körper notwendigerweise durch die erste Öffnung nach innen oder durch die zweite Öffnung nach außen hinaus, da der Durchbruch der Rasteinrichtung keinen ausreichenden Platz zur Aufnahme des gesamten Körpers bietet. Ragt der Körper in dieser Variante durch die zweite Öffnung nach außen, befindet sich die Rasteinrichtung in ihrer Rastposition, da sie über die Außenumhüllende der Außenteilwandung hinausragt. Ragt der Körper hingegen durch die erste Öffnung zum Innenteil des Adapters, befindet sich die Rasteinrichtung in ihrer Freigabeposition, da sie dann nicht mehr über die Außenumhüllende der Außenteilwandung hinausragt.

In einer Ausgestaltung umfasst die Relativbewegung zwischen dem Außenteil und dem Innenteil des Adapters, durch die der Adapter von der Freigabeposition in die Rastposition oder von der Rastposition in die Freigabeposition überführt wird, eine Bewegung entlang der Längserstreckungsachse. Alternativ handelt es sich bei der Relativbewegung zwischen dem Außenteil und dem Innenteil ausschließlich um eine Bewegung entlang der Längserstreckungsachse. In beiden Fällen führt die Bewegung entlang der Längserstreckungsachse dazu, dass der Körper im Durchbruch der Rasteinrichtung zwangsgeführt wird und die Rasteinrichtung von der Rastposition in die Freigabeposition bzw. von der Freigabeposition in die Rastposition überführt. Das heißt, die Längsbewegung zwischen dem Außenteil und dem Innenteil entlang der Längserstreckungsachse wird durch die spezifische Ausgestaltung des Außenteils und des Innenteils in eine Radialbewegung des Körpers der Rasteinrichtung umgesetzt.

In einer Ausgestaltung weist der Adapter ein Verriegelungselement auf, dass relativ zum Außenteil und zum Innenteil zwischen einer Entriegelungsposition und einer Verriegelungsposition beweglich ist. Dabei gestattet das Verriegelungselement in der Entriegelungsposition die Relativbewegung zwischen dem Außenteil und dem Innenteil. Demgegenüber verhindert das Verriegelungselement in der Verriegelungsposition diese Relativbewegung zwischen dem Außenteil und dem Innenteil. Somit kann über das Verriegelungselement gesteuert werden, ob die Rasteinrichtung von der Freigabeposition in die Rastposition bzw. von der Rastposition in die Freigabeposition überführt werden kann. Denn nur wenn sich das Verriegelungselement in seiner Entriegelungsposition befindet, kann die zur Überführung der Rasteinrichtung von der einen in die andere Position erforderliche Relativbewegung zwischen dem Außenteil und dem Innenteil des Adapters ausgeführt werden.

Das Verriegelungselement kann beispielsweise als Verriegelungsclip ausgestaltet sein.

In einer Ausgestaltung ist das Verriegelungselement quer zur Längserstreckungsachse zwischen der Entriegelungsposition und der Verriegelungsposition beweglich. In dieser Ausgestaltung ist eine Bewegung des Verriegelungselements auf besonders einfache Weise unabhängig von einem auf den Adapter bzw. auf den Adapter mit angeschlossenem Hohlkörperobjekt ausgeübten Spüldruck. Denn ein solcher Spüldruck wirkt üblicherweise entlang der Längserstreckungsrichtung des Adapters. Wenn eine Bewegung des Adapters in Längserstreckungsrichtung durch eine quer dazu verlaufende Bewegung des Verriegelungselements aber verhindert wird, kann auch ein hoher Spüldruck nicht zu einem unbeabsichtigten Lösen des Hohlkörperobjekts vom Adapter führen, da der Spüldruck keinen Einfluss auf eine Bewegung des Verriegelungselements quer zur Längserstreckungsrichtung des Adapters hat.

In einer Ausgestaltung ist das Verriegelungselement mit einem ersten Vorspannelement in die Verriegelungsposition vorgespannt. Das heißt, das Verriegelungselement wird durch das erste Vorspannelement automatisch in die Verriegelungsposition überführt, wenn auf das Verriegelungselement keine Kraft ausgeübt wird, die einer von dem ersten Vorspannelement erzeugten Vorspannkraft entgegengewirkt. Auf diese Weise kann eine besonders sichere und dauerhafte Verbindung zwischen dem Adapter und einem daran angeschlossenen Hohlkörperobjekt sichergestellt werden. Denn um ein derart mit dem Adapter verbundenes Hohlkörperobjekt vom Adapter zu lösen, muss zuerst das Verriegelungselement entgegen der von dem ersten Vorspannelement aufgebrachten Vorspannkraft in die Entriegelungsposition überführt werden. Anschließend kann eine Relativbewegung zwischen dem Außenteil und dem Innenteil des Adapters verfolgen, durch die die Rasteinrichtung von der Rastposition in die Freigabeposition überführt wird und das Hohlkörperobjekt vom Adapter entfernt werden kann.

In einer Variante ist das erste Vorspannelement als Druckfeder ausgestaltet. In einer anderen Variante ist das erste Vorspannelement als Zugfeder ausgestaltet.

In einer Ausgestaltung umfasst die Relativbewegung zwischen dem Außenteil und dem Innenteil, die zur Überführung der Rasteinrichtung von der Freigabeposition in die Rastposition bzw. von der Rastposition in die Freigabeposition erforderlich ist, eine Bewegung radial um die Längserstreckungsachse des Adapters herum. In einer weiteren Variante handelt es sich bei der Relativbewegung zwischen dem Außenteil und dem Innenteil ausschließlich um eine Bewegung radial um die Längserstreckungsachse herum. In einer Variante umfasst die Relativbewegung zwischen dem Außenteil und dem Innenteil eine Bewegung, die sowohl Anteile radial um die Längserstreckungsachse herum als auch Anteile entlang der Längserstreckungsachse umfasst. Relevant ist lediglich, dass überhaupt eine Relativbewegung zwischen dem Außenteil und dem Innenteil des Adapters erfolgt, um die Rasteinrichtung von der Freigabeposition in die Rastposition bzw. von der Rastposition in die Freigabeposition zu überführen.

In einer Variante weist das Innenteil eine zum Außenteil orientierte Querschnittsverjüngung auf. Diese Querschnittsverjüngung fluchtet in der Freigabeposition der Rasteinrichtung mit dem Durchbruch. Dann kann der Körper in die Querschnittsverjüngung hineinragen. Bei der Querschnittsverjüngung kann es sich beispielsweise um eine Ausnehmung auf der Außenseite des Innenteils handeln. Durch eine solche Querschnittsverjüngung bzw. Ausnehmung wird ein Raum geschaffen, in den der Körper hineinragen kann, wenn der Durchbruch mit der Querschnittsverjüngung fluchtet. Befinden sich der Durchbruch und die Querschnittsverjüngung des Innenteils hingegen an unterschiedlichen Positionen, ist ein solches Hineinragen des Körpers in die Querschnittsverjüngung nicht möglich. Der Körper kann dann eine Rastkraft zwischen dem Adapter und einem daran angeschlossenen Hohlkörperobjekt aufbringen, sodass sich die Rasteinrichtung in der Rastposition befindet.

In einer Ausgestaltung fluchtet die Querschnittsverjüngung in der Rastposition stets nicht mit dem Durchbruch. Dann ist sichergestellt, dass der Körper in der Rastposition nicht in die Querschnittsverjüngung eintreten kann.

Es ist nicht erforderlich, dass der Körper in der Freigabeposition unter Vorspannung in dem Durchbruch gelagert ist. Vielmehr ist es denkbar und in einer Ausgestaltung vorgesehen, dass der Körper in der Freigabeposition vorspannungsfrei in dem Durchbruch gelagert ist. Es ist somit nicht erforderlich, den Körper durch spezifische Vorspannungselemente wie Federn in einer bestimmten Position in dem Durchbruch zu halten, wenn sich die Rasteinrichtung des Adapters in der Freigabeposition befindet. Dies erleichtert die Konstruktion des Adapters und verlängert dessen Lebensdauer, da es nicht erforderlich ist, über einen langen Zeitraum eine definierte Vorspannung des Körpers im Durchbruch aufrechtzuerhalten.

In einer Ausgestaltung ist die als Sprengring ausgestaltete Bewegungsbegrenzungseinrichtung in der Freigabeposition der Rasteinrichtung vorspannungsfrei in dem Durchbruch gelagert. So wird in dieser Variante nur dann eine Vorspannung auf den Sprengring ausgeübt, wenn sich die Rasteinrichtung in der Rastposition befindet. Denn dann drückt der Körper den Sprengring radial nach außen, damit er in eine Nut eines zu kontaktierenden Hohlkörperobjekts hineinragen kann und dieses Hohlkörperobjekt mittels Formschluss an dem Adapter fixieren kann.

Grundsätzlich ist es denkbar, eine beliebige Anzahl von Körpern in dem Durchbruch zu lagern. In einer Variante ist es allerdings vorgesehen, dass genau ein einziger Körper in dem Durchbruch gelagert ist. Dies reduziert den Aufwand bei der Herstellung des Adapters. Denn wenn mehr als ein Körper in dem Durchbruch gelagert werden soll, muss bei der Montage darauf geachtet werden, dass die in dem Durchbruch befindlichen Körper in der beabsichtigten Art und Weise miteinander zusammenwirken. Ist hingegen nur ein einzelner Körper in dem Durchbruch gelagert, kommt es auf ein solches Zusammenwirken nicht an. Vielmehr kann dieser einzige Körper dann selbst als Rastelement dienen oder einen Sprengring nach außen drücken, damit er zusammen mit dem Sprengring als Rastelement fungiert. Mitunter ist es beim Einsatz mehrerer Körper im Durchbruch sogar erforderlich, bestimmte Geometrien oder Dimensionen der unterschiedlichen Körper, die in ein und demselben Durchbruch gelagert sind, vorzusehen. Dies ist naturgemäß nicht erforderlich, wenn lediglich ein einziger Körper in dem Durchbruch gelagert ist und mit der Bewegungsbegrenzungseinrichtung zusammenwirkt, um die Rasteinrichtung von der Freigabeposition in die Rastposition bzw. von der Rastposition in die Freigabeposition zu überführen.

Der Adapter kann bereits mit einer einzigen Rasteinrichtung eine sichere Fixierung eines Hohlkörperobjekts an dem Adapter gewährleisten. Für eine besonders sichere und dauerhafte Befestigung eines Hohlkörperobjekts an dem Adapter ist es indes vorteilhaft, wenn der Adapter - wie in einer Ausgestaltung vorgesehen - mindestens zwei Rasteinrichtungen aufweist. Diese zwei oder mehr Rasteinrichtungen können grundsätzlich unregelmäßig entlang einer Umfangsrichtung des Adapters realisiert sein. In einer Variante ist es allerdings vorgesehen, dass die zwei oder mehr Rasteinrichtungen gleichmäßig entlang einer Umfangsrichtung des Außenteils im Außenteil angeordnet sind. Wenn beispielsweise genau zwei Rasteinrichtungen vorgesehen sind, ist es vorteilhaft, wenn sich diese gegenüberliegen, also in einem Winkel von 180° zueinander angeordnet sind. Wenn drei Rasteinrichtungen vorgesehen sind, ist es vorteilhaft, wenn diese in einem Winkel von jeweils 120° zueinander angeordnet sind, sich also in einem gleichmäßigen Abstand entlang der Umfangsrichtung des Außenteils verteilen. Bei vier Rasteinrichtungen würde man vorzugsweise einen Winkelabstand von 90° zwischen den einzelnen Rasteinrichtungen vorsehen. Grundsätzlich ist es somit angedacht, n Rasteinrichtungen (mit n größer gleich 2) in einem Winkel von jeweils 360°/n zueinander anzuordnen.

Damit das Außenteil und das Innenteil besonders einfach relativ zueinander bewegt werden können und damit so die Rasteinrichtung besonders einfach von der Rastposition in die Freigabeposition bzw. von der Freigabeposition in die Rastposition überführt werden kann, sind das Außenteil und das Innenteil in einer Variante durch ein zweites Vorspannelement gegeneinander in eine relative Positionierung vorgespannt, in der die Rasteinrichtung des Adapters in der Freigabeposition vorliegt. Wenn grundsätzlich eine Bewegung zwischen dem Außenteil und dem Innenteil des Adapters möglich ist (beispielsweise, weil sich das Verriegelungselement in seiner Entriegelungsposition befindet), sorgt das zweite Vorspannelement dann dafür, dass das Außenteil und das Innenteil derart zueinander bewegt werden, dass die Rasteinrichtung in die Freigabeposition überführt wird. Ein Hohlkörperobjekt kann dann besonders einfach von dem Adapter entfernt werden bzw. auf den Adapter aufgesteckt werden. Zur sicheren Kontaktierung eines Hohlkörperobjekts an dem Adapter ist es lediglich erforderlich, eine Kraft entgegen der von dem zweiten Vorspannelement ausgeübten Vorspannkraft auszuüben, damit eine Relativbewegung zwischen dem Außenteil und dem Innenteil entgegen dieser Vorspannkraft erfolgt, wodurch dann die Rasteinrichtung des Adapters von der Freigabeposition in die Rastposition überführt wird.

Ein Aspekt der vorliegend beanspruchten Erfindung betrifft eine Adapteranordnung, die einen Adapter gemäß den vorherigen Erläuterungen und ein auf einer Außenseite des Außenteils des Adapters angeordnetes Hohlkörperobjekt umfasst. Dieses Hohlkörperobjekt ist in der Freigabeposition der Rasteinrichtung lösbar mit dem Adapter verbunden und in der Rastposition der Rasteinrichtung i) formschlüssig oder ii) form- und kraftschlüssig oder iii) kraftschlüssig mit dem Adapter verbunden. Auf diese Weise wird in der Rastposition der Rasteinrichtung eine sichere Verbindung zwischen dem Adapter und dem Hohlkörperobjekt realisiert, die erst bei Überführung der Rasteinrichtung in die Freigabeposition gelöst werden kann. In der Freigabeposition lässt sich das Hohlkörperobjekt dann leicht von dem Adapter entfernen.

In einer Ausgestaltung weist das Hohlkörperobjekt eine radial innenliegende Nut auf. Diese radial innenliegende Nut dient dazu, dass die Rasteinrichtung (insbesondere mittels des Körpers der Rasteinrichtung bzw. mittels der als Sprengring ausgestalteten Bewegungsbegrenzungseinrichtung) in die Nut des Hohlkörperobjekts eingreift und so die i) formschlüssige oder ii) form- und kraftschlüssige oder iii) kraftschlüssige Verbindung zwischen dem Adapter und dem Hohlkörperobjekt realisiert.

Ein Aspekt der vorliegend beanspruchten Erfindung betrifft ein Verfahren zum Verbinden eines Hohlkörperobjekts mit einem Gerät zur Innendurchspülung von Hohlkörperobjekten und zum Lösen des Hohlkörperobjekts von dem Gerät. Dieses Verfahren ist durch die nachfolgend erläuterten Schritte gekennzeichnet.

Zunächst wird ein Hohlkörperobjekt auf einer Außenseite eines Adapters gemäß den vorherigen Erläuterungen aufgesteckt. Anschließend erfolgt eine relative Bewegung des Außenteils und des Innenteils des Adapters zueinander. Dadurch wird die Rasteinrichtung des Adapters von ihrer Freigabeposition in ihre Rastposition überführt.

Zu einem späteren Zeitpunkt erfolgt abermals eine relative Bewegung des Außenteils und des Innenteils des Adapters zueinander (aber in genau der entgegengesetzten Richtung als zuvor), um die Rasteinrichtung von der Rastposition wieder in die Freigabeposition zu überführen. In dieser Freigabeposition kann das Hohlkörperobjekt dann von der Außenseite des Adapters entfernt werden.

Typischerweise erfolgt in dem Zeitraum, in dem das Hohlkörperobjekt auf dem Adapter angeordnet ist und mittels der Rasteinrichtung in deren Rastposition sicher mit dem Adapter verrastet ist, eine Reinigung und/oder Desinfektion des Hohlkörperobjekts. Zu diesem Zweck wird ein geeignetes Spülmedium durch ein Innenlumen des Hohlkörperobjekts geleitet. Dabei wird das Spülmedium durch einen in dem Adapter ausgebildeten Kanal zu dem zu reinigenden Hohlkörperobjekt geführt. Darüber hinaus kann über einen gesonderten Medienkanal, der ebenfalls in dem Adapter ausgebildet ist, ein Pflegemittel wie etwa ein Dentalöl dem Hohlkörperobjekt zugeführt werden. Dies ist insbesondere dann sinnvoll, wenn das Hohlkörperobjekt einen zu pflegenden Antriebskanal aufweist, in dem sich bewegliche Teile befinden, die regelmäßig mittels eines Öls gepflegt werden sollen.

Eine Überführung der Rasteinrichtung von der Rastposition in die Freigabeposition erfolgt regelmäßig dann, wenn die Reinigung und/oder Desinfektion vollständig abgeschlossen ist. Typischerweise umfasst die Reinigung und/oder Desinfektion auch eine Trocknung des Hohlkörperobjekts, bei der heiße trockene Luft und/oder Druckluft durch das Hohlkörperobjekt hindurch und/oder auf eine Außenseite des Hohlkörperobjekts geleitet wird. Diese Reinigungs- und/oder Desinfektionsschritte sind dem Fachmann grundsätzlich gut bekannt.

Alle Varianten und Ausgestaltungen des Adapters sind in beliebiger Weise miteinander kombinierbar und entweder einzeln oder in beliebiger Kombination auf die Adapteranordnung und auf das Verfahren übertragbar. In gleicher Weise sind alle Varianten und Ausgestaltungen der Adapteranordnung in beliebiger Weise miteinander kombinierbar und entweder einzeln oder in beliebiger Kombination auf den Adapter oder das Verfahren übertragbar. Schließlich können alle Varianten und Ausgestaltungen des Verfahrens in beliebiger Weise miteinander kombiniert werden und entweder einzeln oder in beliebiger Kombination auf den Adapter oder auf die Adapteranordnung übertragen werden.

Weitere Details von Aspekten der vorliegenden Erfindung werden nachfolgend anhand von Ausführungsbeispielen und Figuren näher erläutert. Es zeigen:
- Figur 1A: einen Längsschnitt durch ein erstes Ausführungsbeispiel einer Adapteranordnung in ihrer Freigabeposition;
- Figur 1B: eine vergrößerte Detailansicht der Adapteranordnung der Figur 1A;
- Figur 1C: eine Querschnittsansicht entlang der Linie C in der Figur 1B;
- Figur 1D: einen Längsschnitt durch die Adapteranordnung der Figur 1A in ihrer Rastposition;
- Figur 1E: eine vergrößerte Detailansicht der Adapteranordnung der Figur 1D;
- Figur 1F: eine Querschnittsansicht entlang der Linie F in der Figur 1E;
- Figur 1G: eine Querschnittsansicht entlang der Linie G in der Figur 1B;
- Figur 2A: einen Längsschnitt durch ein zweites Ausführungsbeispiel einer Adapteranordnung in ihrer Freigabeposition;
- Figur 2B: eine vergrößerte Detailansicht der Adapteranordnung der Figur 2A;
- Figur 2C: eine Querschnittsansicht entlang der Linie C in der Figur 2B;
- Figur 2D: einen Längsschnitt durch die Adapteranordnung der Figur 2A in ihrer Rastposition;
- Figur 2E: eine vergrößerte Detailansicht der Adapteranordnung der Figur 2D;
- Figur 2F: eine Querschnittsansicht entlang der Linie F in der Figur 2E;
- Figur 3A: eine Detailansicht durch einen Längsschnitt eines dritten Ausführungsbeispiels einer Adapteranordnung in ihrer Freigabeposition;
- Figur 3B: eine Querschnittsansicht entlang der Linie B in der Figur 3A;
- Figur 3C: eine Querschnittsansicht entlang der Linie C in der Figur 3A in der Freigabeposition der Adapteranordnung;
- Figur 3D: die Querschnittsansicht der Figur 3C in der Rastposition der Adapteranordnung;
- Figur 4A: eine perspektivische Ansicht eines vierten Ausführungsbeispiels eines Adapters in dessen Freigabeposition;
- Figur 4B: eine perspektivische Ansicht des Adapters der Figur 4A in dessen Rastposition;
- Figur 4C: den Adapter der Figur 4A in einer Seitenansicht;
- Figur 4D: den Adapter der Figur 4C in einer Längsschnittansicht;
- Figur 4E: eine Querschnittsansicht durch den Adapter der Figur 4C entlang der Linie E;
- Figur 4F: eine Querschnittsansicht durch den Adapter der Figur 4C entlang der Linie F;
- Figur 4G: den Adapter der Figur 4B in einer Seitenansicht;
- Figur 4H: den Adapter der Figur 4G in einer Längsschnittansicht;
- Figur 4I: eine Querschnittsansicht durch den Adapter der Figur 4G entlang der Linie I;
- Figur 4J: eine Querschnittsansicht durch den Adapter der Figur 4G entlang der Linie J; und
- Figur 5: eine Längsschnittansicht eines fünften Ausführungsbeispiels einer Adapteranordnung in deren Rastposition.

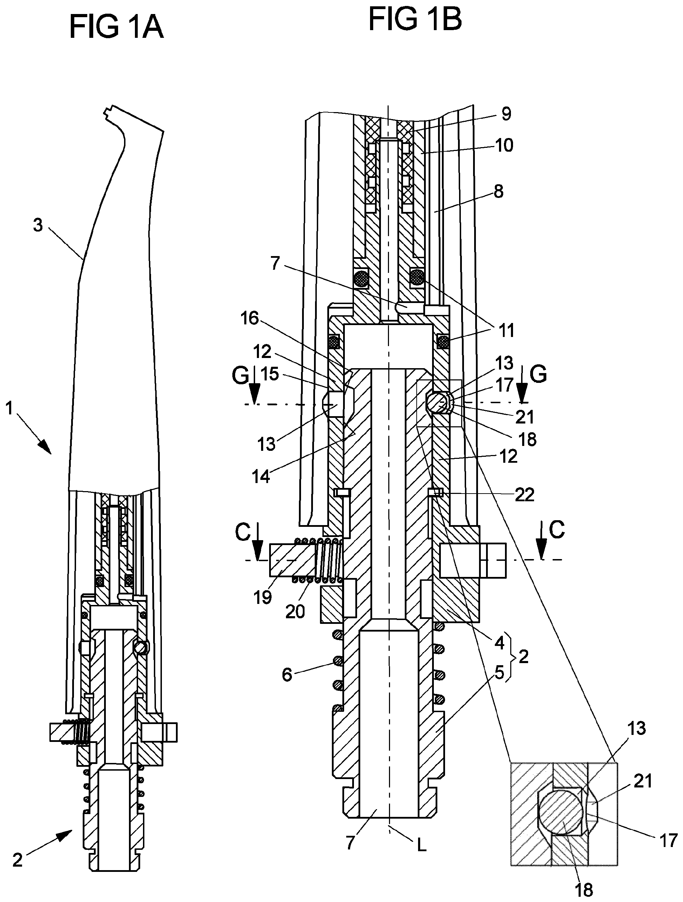

Die Figur 1 A zeigt eine Längsschnittansicht durch eine Adapteranordnung 1 bestehend aus einem Adapter 2 und einem auf den Adapter 2 aufgesteckten Hohlkörperinstrument 3, das als Hohlkörperobjekt dient. Das Hohlkörperinstrument 3 ist in dem in der Figur 1A dargestellten Zustand der Adapteranordnung 1 nicht fest mit dem Adapter 2 verbunden. Vielmehr liegt der Adapter 2 in seiner entkoppelten Position oder Freigabeposition vor. Der genaue Aufbau des Adapters 2 wird unter Bezugnahme auf die Figur 1B näher erläutert. Dabei werden in allen Figuren vergleichbare Elemente mit denselben Bezugszeichen versehen. Ferner ist zu beachten, dass die Figuren nicht maßstabsgetreu ausgeführt sind und nicht alle technischen Einzelheiten der beschriebenen Objekte mit dem gleichen Detaillierungsgrad wiedergegeben werden. Soweit ein technisches Detail für den beschriebenen Gegenstand eine besondere Bedeutung hat, wird darauf bei der Figurenbeschreibung gesondert hingewiesen und/oder das entsprechende Detail vergrößert bzw. gesondert dargestellt.

Die Figur 1B zeigt eine Detaildarstellung der Längsschnittansicht der Figur 1A. Der Adapter 2 weist ein Außenteil 4 und ein Innenteil 5 auf, die beweglich zueinander gelagert sind. Dabei wird das Außenteil 4 durch eine Druckfeder 6 gegenüber dem Innenteil 5 in die in der Figur 1B dargestellte Freigabeposition vorgespannt. Entlang einer Längserstreckungsachse L des Adapters 2 sowie quer zur Längserstreckungsachse L ist sowohl im Innenteil 5 als auch im Außenteil 4 ein Adapterkanal 7 ausgebildet, durch den ein Spülmedium in einen mit dem Adapterkanal 7 verbundenen Instrumentenkanal 8 des Hohlkörperinstruments 3 geleitet werden kann. Damit ein solches Spülmedium nicht unbeabsichtigt aus dem Adapterkanal 7 oder dem Instrumentenkanal 8 austritt, ist zwischen dem Adapter 2 und dem Hohlkörperinstrument 3 ein Silikonformteil 9 angeordnet, das durch eine Haltehülse 10 in Position gehalten wird. Das Silikonformteil 9 sorgt für eine dichtende Verbindung zwischen dem Adapterkanal 7 und dem Instrumentenkanal 8. Darüber hinaus sind noch 2 O-Ringe 11 zwischen dem Außenteil 4 und einer Innenseite des Hohlkörperinstruments 3 angeordnet, die ebenfalls für eine Abdichtung zwischen dem Hohlkörperinstrument 3 und dem Adapter 2 sorgen.

Das Außenteil 4 weist eine Außenteilwandung 12 auf, in der sechs Durchbrüche 13 ausgebildet sind, von denen zwei Durchbrüche 13 in der Längsschnittdarstellung der Figur 1 B zu sehen sind.. Die Durchbrüche 13 erstrecken sich dabei jeweils von einer Innenseite 14 der Außenteilwandung 12 zu einer Außenseite 15 der Außenteilwandung 12. In der Innenseite 14 ist bei jedem Durchbruch 13 eine erste Öffnung 16 realisiert, während in der Außenseite 15 bei jedem Durchbruch 13 eine zweite Öffnung 17 realisiert ist. Im rechten Durchbruch 13 ist eine Kugel 18 beweglich gelagert. Die erste Öffnung 16 ist derart dimensioniert, dass die Kugel 18 durch die erste Öffnung 16 zum Innenteil 5 des Adapters 2 hindurchtreten kann. Das Innenteil 5 weist in der Freigabeposition auf Höhe des Durchbruchs 13 eine Aussparung auf, in die die Kugel 18 hineinragen kann. Dies ist in der als Einsatz in der Figur 1B wiedergegebenen vergrößerten Darstellung der Rasteinrichtung sichtbar.

Die zweite Öffnung 17 des rechten Durchbruchs 13 ist demgegenüber etwas kleiner dimensioniert, sodass die Kugel 18 nicht vollständig durch die zweite Öffnung 17 nach außen treten kann (vergleiche hierzu ebenfalls die als Einsatz in der Figur 1B wiedergegebene vergrößerte Darstellung der Rasteinrichtung). Die Kugel 18 kann jedoch partiell durch die zweite Öffnung 17 über die Außenseite 15 der Außenteilwandung 12 hinausragen. Dies ist in der in der Figur 1B dargestellten Freigabeposition jedoch nicht der Fall. Hier wird die Kugel 18 vielmehr in der am Innenteil 5 vorgesehenen Ausnehmung aufgenommen. Aus fertigungstechnischen Gründen ist die zweite Öffnung 17 des linken Durchbruchs 13 gleich groß wie die erste Öffnung 16 des linken Durchbruchs 13 bzw. des rechten Durchbruchs 13 (die Durchbrüche 13 wurden in diesem Ausführungsbeispiel durch eine von der linken Seite erfolgende Bohrung erzeugt).

Der Durchbruch 13 mit seiner ersten Öffnung 16 und einer zweiten Öffnung 17 bildet zusammen mit der Kugel 18 eine Rasteinrichtung. In dem Betriebszustand des Adapters 2, der in der Figur 1B dargestellt ist, befindet sich diese Rasteinrichtung in ihrer Freigabeposition. In dieser Freigabeposition ist es problemlos möglich, das Hohlkörperinstrument 3 auf den Adapter 2 aufzusetzen und von dem Adapter 2 zu entfernen. Die Rasteinrichtung verhindert eine solche Bewegung nicht.

Der Adapter 2 weist zudem einen Verriegelungsclip 19 auf, der zwischen einer Verriegelungsposition und einer Entriegelungsposition beweglich gelagert ist und mit einer zweiten Feder 20 in die Verriegelungsposition vorgespannt ist. In dem in der Figur 1B dargestellten Betriebszustand des Adapters 2 ist der Verriegelungsclip 19 bereits durch Ausüben einer Kraft, die der von der zweiten Feder 20 aufgebrachten Vorspannkraft entgegengerichtet ist, in seine Entriegelungsposition überführt.

Ein Sicherungsring 22 begrenzt eine nach oben gerichtete Relativbewegung zwischen dem Außenteil 4 und dem Innenteil 5 des Adapters 2. Das heißt, dass die von der Druckfeder 6 aufgebrachte Federkraft nicht zu einem Auseinanderfallen des Adapters 2 führt.

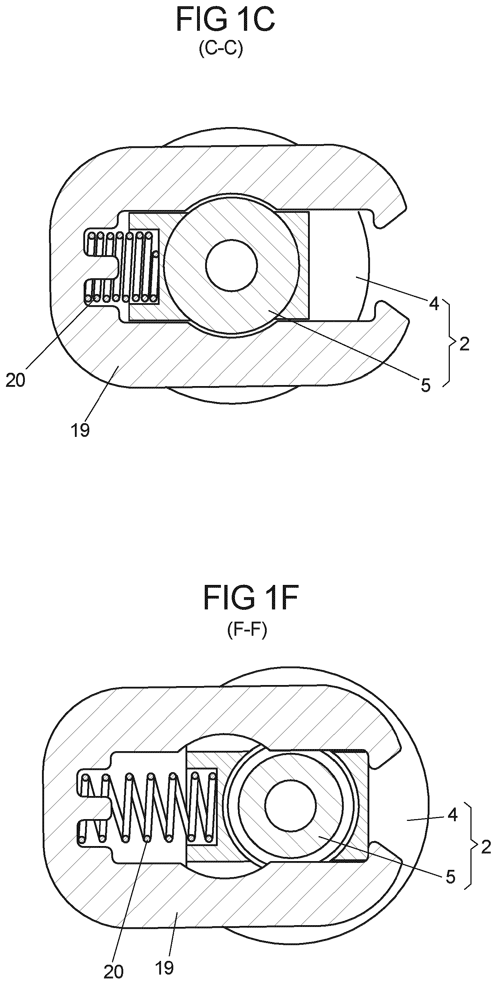

Der Verriegelungsclip 19 ist in der Figur 1C in einer entlang der Linie C der Figur 1B ausgeführten Querschnittsansicht gesondert dargestellt.

Der Verriegelungsclip 19 hat eine Form, mit der er eine Bewegung des Innenteils 5 relativ zum Außenteil 4 des Adapters 2 ermöglicht, wenn er sich in der in der Figur 1C dargestellten Entriegelungsposition befindet.

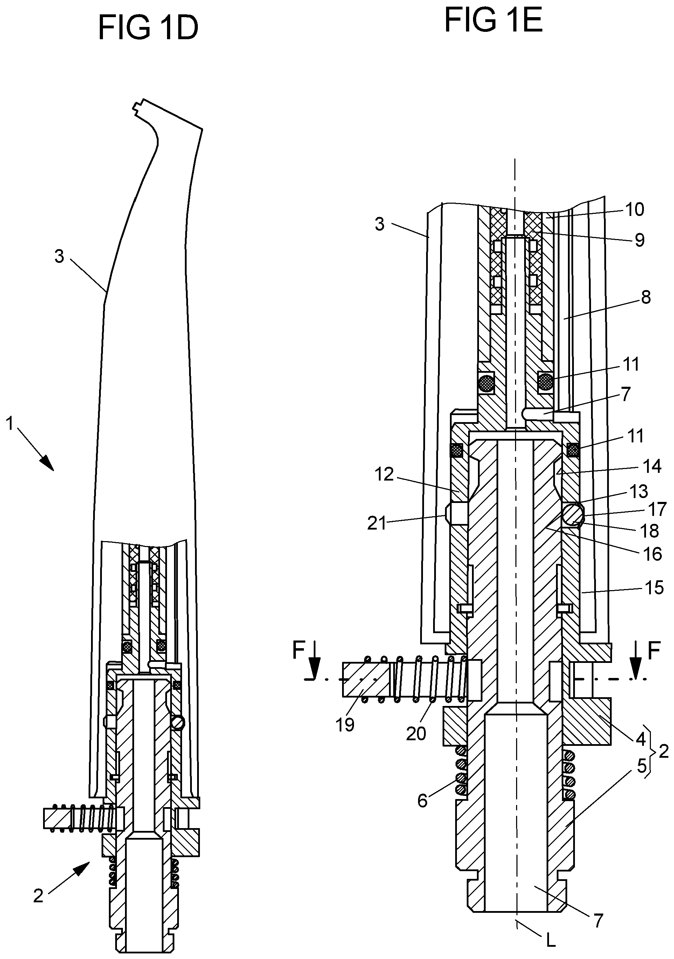

Die Figur 1D zeigt die Adapteranordnung 1 der Figur 1A in ihrem zweiten Betriebszustand, nämlich in ihrer Rastposition, in der das Hohlkörperinstrument 3 fest mit dem Adapter 2 verbunden ist. Dies ist in der vergrößerten Detaildarstellung der Figur 1E noch genauer zu erkennen.

Um den Adapter 2 von der Freigabeposition in die Rastposition zu überführen, wird das Hohlkörperinstrument 3 entgegen der von der Druckfeder 6 aufgebrachten Federkraft auf das Außenteil 4 gedrückt. Dadurch bewegt sich das Außenteil 4 nach unten, während sich die Position des Innenteils 5 nicht verändert. Es kommt also zu einer Relativbewegung zwischen dem Außenteil 4 und dem Innenteil 5. Dadurch fluchten die Ausnehmung am Innenteil 5 und der Durchbruch 13 (und damit die im Durchbruch 13 befindliche Kugel 18) nicht mehr miteinander. Vielmehr drückt die Außenkontur des Innenteils 5 die Kugel 18 radial nach außen. Da die zweite Öffnung 17 jedoch ein vollständiges Hindurchtreten der Kugel 18 verhindert, kann diese lediglich mit einem geringen Bereich über die Außenumhüllende der Außenteilwandung 12 des Außenteils 4 des Adapters 2 hinausragen. Dadurch wird jedoch einer Rastkraft gegenüber dem Hohlkörperinstrument 3 aufgebracht. Zudem ist in dem Hohlkörperinstrument 3 eine Nut 21 ausgebildet, in die die Kugel 18 hineinragt. Dadurch wird ein Formschluss zwischen dem Adapter 2 und dem Hohlkörperinstrument 3 realisiert. Zugleich wird der Verriegelungsclip 19 durch die zweite Feder 20 in seine Verriegelungsposition gedrückt.

Diese Verriegelungsposition ist in dem entlang der Linie F dargestellten Querschnitt des Verriegelungsclips 19 in der Figur 1F im Detail gezeigt. Durch die Konturierung des Verriegelungsclips 19 wird eine Relativbewegung zwischen dem Außenteil 4 und dem Innenteil 5 des Adapters 2 in der Verriegelungsposition des Verriegelungsclips 19 verhindert. Vielmehr ist eine solche Relativbewegung zwischen dem Außenteil 4 und dem Innenteil 5 erst dann wieder möglich, wenn der Verriegelungsclip 19 in die Entriegelungsposition überführt wird, die in der Figur 1C dargestellt ist.

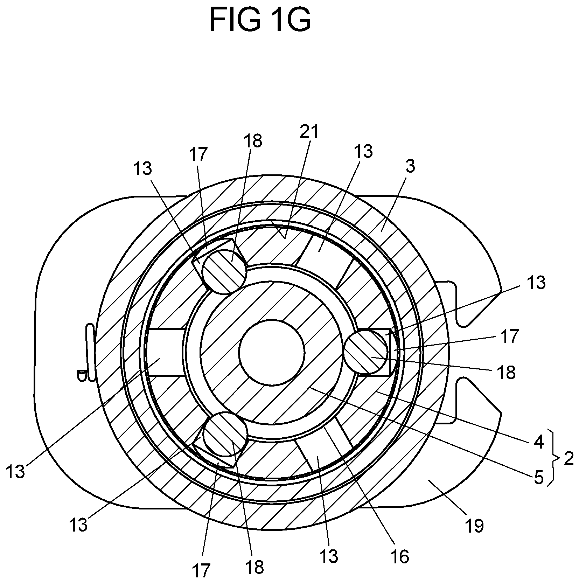

Die genaue Anordnung und Ausgestaltung der Durchbrüche 13 ist aus der entlang der Linie G-G in der Figur 1B wiedergegebenen Querschnittsdarstellung der Figur 1G ersichtlich. Jeweils zwei Durchbrüche 13 liegen sich gegenüber und werden in einem Fertigungsschritt hergestellt. Dazu erfolgt eine Bohrung, die sich vollständig durch einen ersten der sich gegenüberliegenden Durchbrüche 13 erstreckt, die im zweiten der sich gegenüberliegenden Durchbrüche jedoch nicht bis vollständig auf die Außenseite (zur radial innenliegenden Nut 21 des Hohlkörperinstruments 3 hin) ausgeführt wird. Vielmehr wird durch die nicht vollständige Bohrung im zweiten der sich gegenüberliegenden Durchbrüche 13 die zweite Öffnung 17 gebildet, die einen Öffnungsdurchmesser aufweist, der etwas kleiner als der Durchmesser der Kugel 18 ist. Dadurch kann die Kugel 18 in ihrer Rastposition zwar teilweise durch die zweite Öffnung 17 radial nach außen gedrückt werden und in die Nut 21 des Hohlkörperinstruments 3 eingreifen. Sie kann durch die zweite Öffnung 17 jedoch nicht aus dem Durchbruch 13 herausfallen, wenn kein Hohlkörperinstrument 3 mit dem Adapter 2 verbunden ist.

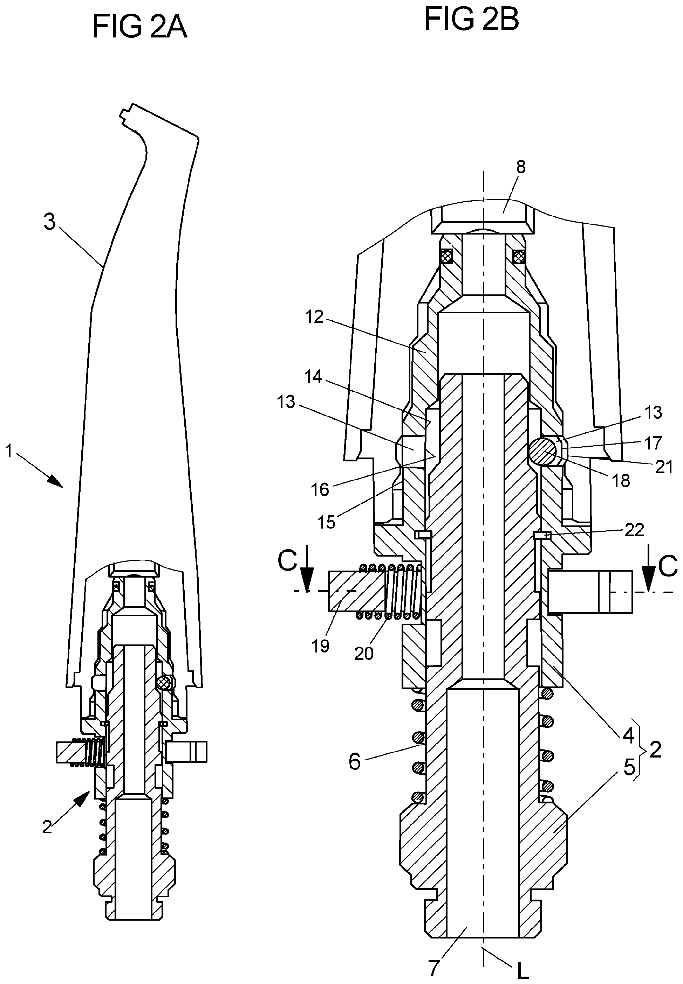

Die Figur 2A zeigt eine Längsschnittansicht durch ein zweites Ausführungsbeispiel einer Adapteranordnung 1 mit einem Adapter 2 und einem Hohlkörperinstrument 3. Das Hohlkörperinstrument 3 weist eine andere Außen- und Innengeometrie auf als das Hohlkörperinstrument 3 des Ausführungsbeispiels, das in den Figuren 1A bis 1F dargestellt ist. Durch eine entsprechend angepasste Außengeometrie des Außenteils 4 des Adapters 2 kann aber auch ein derart ausgestaltetes Hohlkörperinstrument 3 problemlos mit dem Adapter 2 verbunden werden. Das Funktionsprinzip der Verbindung ist dabei identisch zu dem Funktionsprinzip, das für die Adapteranordnung 1 der Figuren 1A bis 1F erläutert wurde. Insoweit wird auf die diesbezüglichen obigen Erläuterungen verwiesen.

Die Figur 2B zeigt eine vergrößerte Detaildarstellung des Längsschnitts der Figur 2A. Dabei fällt auf, dass der in Figuren 2A und 2B dargestellte Adapter 2 kein Silikonformstück und keine Haltehülse aufweist. Vielmehr erfolgt die Verbindung zwischen dem Adapterkanal 7 des Adapters 2 und dem Instrumentenkanal 8 des Hohlkörperinstruments 3 ohne entsprechendes Silikonformstück, aber dennoch in fluiddichter Weise.

Wie in der Figur 2B zu sehen ist, befindet sich der Adapter 2 in seiner Freigabeposition, in der die Kugel 18 in eine Aussparung auf der Außenseite des Innenteils 5 des Adapters 2 hineinragt. Damit übt die Kugel 18 keine Rastkraft auf das Hohlkörperinstrument 3 aus, sodass dieses ohne weiteres von dem Adapter 2 entfernt werden kann. Der Verriegelungsclip 19 ist - wie bei dem in der Figur 1B dargestellten Betriebszustand des dortigen Adapters 2 - in seiner Entriegelungsposition, sodass das Außenteil 4 und des Innenteil 5 des Adapters 2 gegeneinander beweglich sind.

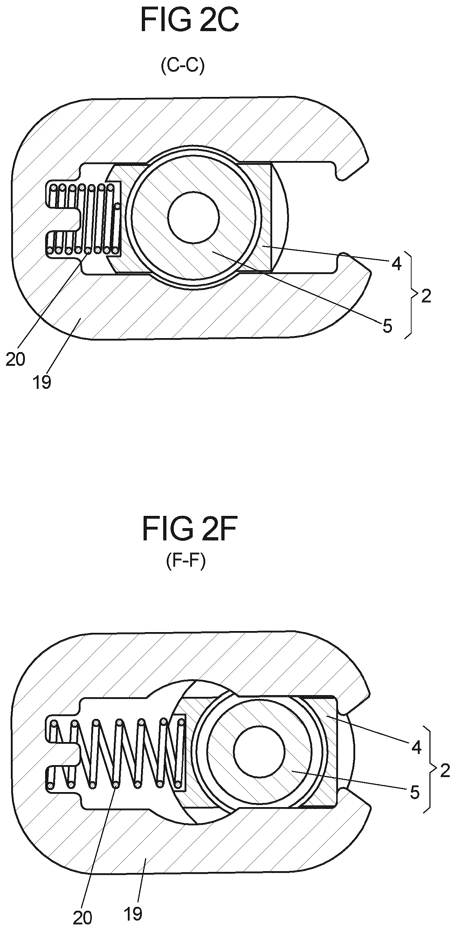

Eine entsprechende Querschnittsansicht entlang der Linie C der Figur 2B ist in der Figur 2C dargestellt. Die Figur 2C ist dabei nahezu identisch zu der Figur 1C, da sich die Querschnitte der Adapter 2 in diesem Bereich nur gering voneinander unterscheiden.

Wie aus einer Zusammenschau der Figuren 2B und 2C gut ersichtlich ist, muss der Verriegelungsclip 19 in einer Richtung bewegt werden, die senkrecht zur Längserstreckungsachse L des Adapters 2 verläuft, um von seiner Entriegelungsposition in seine Verriegelungsposition überführt zu werden. Die zum Lösen des Hohlkörperinstruments 3 aufzubringende Kraft hängt somit maßgeblich von der Federkraft der zweiten Feder 20 ab. Sie steht aber nicht im Verhältnis zu der von dem Adapter 2 über die Rasteinrichtung aufgebrachte Haltekraft gegenüber dem Hohlkörperinstrument 3. Dadurch wird die Lösekraft von der Haltekraft entkoppelt, was einerseits eine besonders feste Verbindung zwischen dem Adapter 2 und dem Hohlkörperinstrument 3 ermöglicht, andererseits aber auch eine besonders einfache Bedienung des Adapters 2 gestattet.

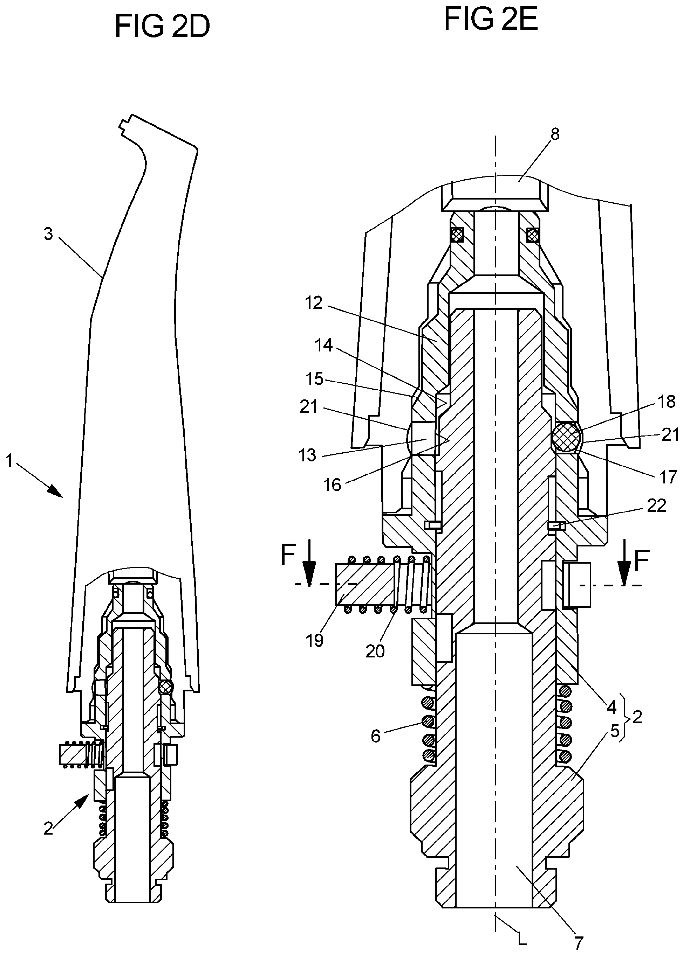

In der Figur 2D ist die Adapteranordnung 1 der Figur 2A in ihrem zweiten Betriebszustand dargestellt, nämlich in der Rastposition des Adapters 2, in der das Hohlkörperinstrument 3 fest mit dem Adapter 2 verbunden ist. Dies ist in der vergrößerten Detaildarstellung der Figur 2E noch genauer zu ersehen.

In der Rastposition drückt die Kontur des Innenteils 5 die Kugel 18 radial nach außen, sodass diese durch die zweite Öffnung 17 teilweise hindurch in die Nut 21 des Hohlkörperinstruments 3 hineinragt und somit für eine formschlüssige Verbindung zwischen dem Hohlkörperinstrument 3 und dem Adapter 2 sorgt.

Zugleich ist der Verriegelungsclip 19 wieder in seiner Verriegelungsposition, die in der Querschnittsdarstellung der Figur 2F entlang der Linie F der Figur 2D dargestellt ist. In dieser Verriegelungsposition verhindert der Verriegelungsclip 19 eine weitere Relativbewegung zwischen dem Innenteil 5 und dem Außenteil 4 des Adapters 2.

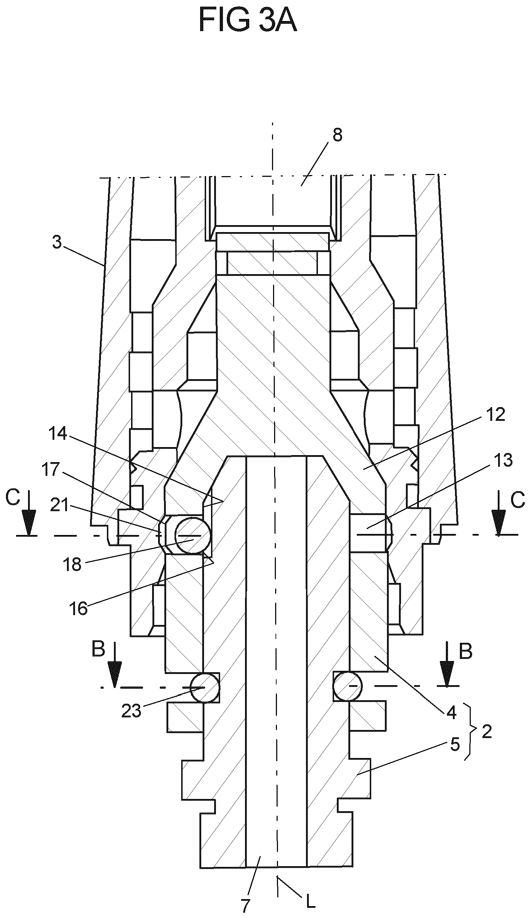

Die Figur 3A zeigt eine Detaildarstellung eines weiteren Ausführungsbeispiels einer Adapteranordnung 1 mit einem Adapter 2 und einem daran gekoppelten Hohlkörperinstrument 3. Wie auch bei den vorherigen Ausführungsbeispielen weist der Adapter 2 ein Außenteil 4 und ein Innenteil 5 auf. Darüber hinaus ist eine Rasteinrichtung vorgesehen, die eine in einem Durchbruch 13 bewegliche Kugel 18 aufweist, welche in ihrer Rastposition das Hohlkörperinstrument 3 an dem Adapter 2 festlegt und in ihrer Freigabeposition ein Aufstecken des Hohlkörperinstruments 3 auf den Adapter 2 ermöglicht bzw. ein Lösen des Hohlkörperinstruments 3 von dem Adapter 2 gestattet. In der Figur 3A ist die Rasteinrichtung in ihrer Freigabeposition dargestellt. Die Kugel 18 ragt dabei über die innere Öffnung 16 des Durchbruchs 13 in eine Ausnehmung zwischen der Innenseite 14 der Außenteilwandung 12 und einer Außenseite des Innenteils 5 hinein. Die Kugel könnte auch in die im Hohlkörperinstrument 3 radial innenliegende Nut 21 hineinragen, würde dort aber keine Rastfunktion ausüben, da sie sich jederzeit frei im Durchbruch 13 zum Innenteil 5 hin bewegen könnte.

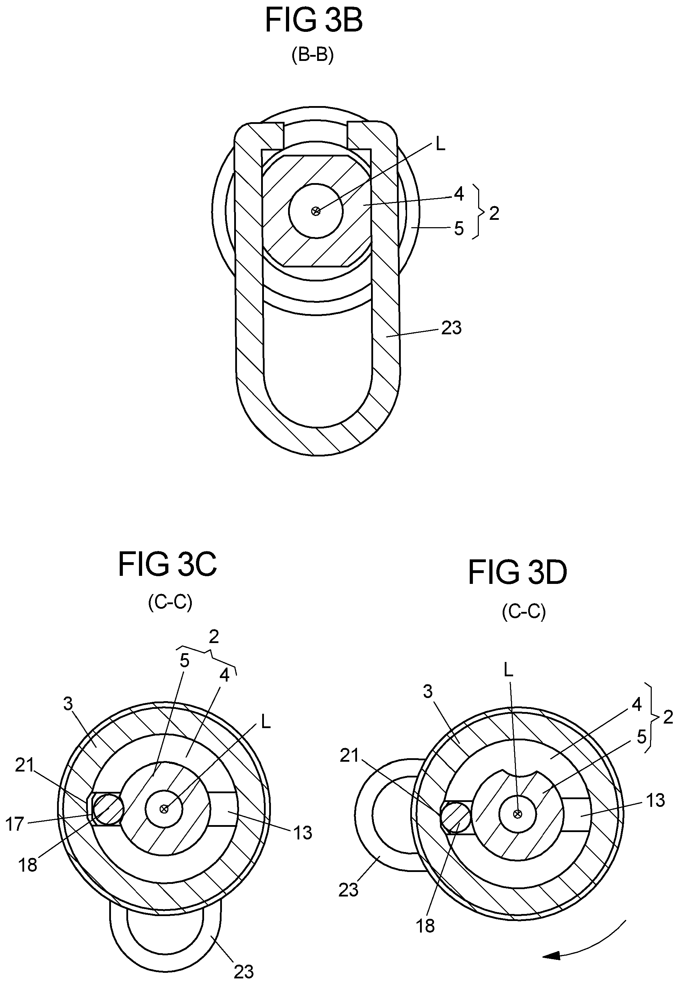

Um die Rasteinrichtung des Adapters 2 von ihrer Freigabeposition in die Rastposition zu überführen, erfolgt bei diesem Ausführungsbeispiel keine Lateralbewegung zwischen dem Außenteil 4 und dem Innenteil 5 des Adapters 2 entlang der Längserstreckungsachse L, sondern eine relative Radialbewegung zwischen dem Außenteil 4 und dem Innenteil 5 um die Längserstreckungsachse L herum. Dies ist in den Figuren 3B bis 3D näher dargestellt.

Dabei zeigt die Figur 3B eine Querschnittsdarstellung entlang der Linie B in der Figur 3A, während Figuren 3C und 3D eine Querschnittsdarstellung entlang der Linie C der Figur 3A in zwei unterschiedlichen Positionen der Adapteranordnung 1 zeigen.

Die Figur 3B zeigt einen Drahtbügel 23, der als Betätigungselement dient. Dieser Drahtbügel 23 steht in Wirkverbindung mit dem Innenteil 5 des Adapters 2. Eine Bewegung des Drahtbügels 23 radial um die Längserstreckungsachse L herum bewirkt eine gleichlaufende Bewegung des Innenteils 5 um die Längserstreckungsachse L herum, während das Außenteil 4 sich dadurch nicht bewegt. Mithin kommt es zu einer relativen Radialbewegung zwischen dem Außenteil 4 und dem Innenteil 5 des Adapters 2.

In der Figur 3C ist eine Schnittdarstellung entlang der mit C in der Figur 3A gekennzeichneten Linie gezeigt. Die Kugel 18 ragt in eine Aussparung auf der Außenseite des Innenteils 5 des Adapters hinein, sodass sie im Durchbruch 13 frei beweglich ist. Wird der Drahtbügel 23 um 90° im Uhrzeigersinn bewegt, bewegt sich die Aussparung von der 9-Uhr-Position in die 12-Uhr-Position. Im Bereich des Durchbruchs 13 befindet sich dann ein aussparungsfreier Abschnitt der Außenseite des Innenteils 5. Dadurch wird die Kugel 18 im Durchbruch 13 zur zweiten Öffnung 17 und gegen eine Innenseite des Hohlkörperinstruments 3 gedrückt. Dadurch wird das Hohlkörperinstrument 3 am Adapter 2 fixiert; die Rasteinrichtung des Adapters 2 befindet sich in der Rastposition.

Die Rasteinrichtung des Adapters 2 kann wieder die Freigabeposition überführt werden, indem der Drahtbügel 23 90° entgegen dem Uhrzeigersinn gedreht wird. Dann fluchtet die Aussparung auf der Außenseite des Innenteils 5 des Adapters 2 wieder mit dem Durchbruch 13, sodass die Kugel 18 nicht mehr aus der zweiten Öffnung 17 herausgedrückt wird, sondern in die Aussparung hineinragen kann. Folglich übt die Kugel 18 dann keine Rastkraft mehr auf das Hohlkörperinstrument 3 aus.

Da das Hohlkörperinstrument 3 im Bereich der Kugel 18 die (in Bezug auf das Hohlkörperinstrument radial innenliegende) Nut 21 aufweist, greift die Kugel 18 in der Rastposition zudem in diese Nut 21 ein und sorgt für eine formschlüssige Verbindung zwischen dem Hohlkörperinstrument 3 und dem Adapter 2. Dadurch ist ein noch sicherer Sitz des Hohlkörperinstruments 3 an dem Adapter 2 möglich, als wenn lediglich auf eine von der Kugel 18 vermittelte Rastkraft zur Festlegung des Hohlkörperinstrument 3 abgestellt wird.

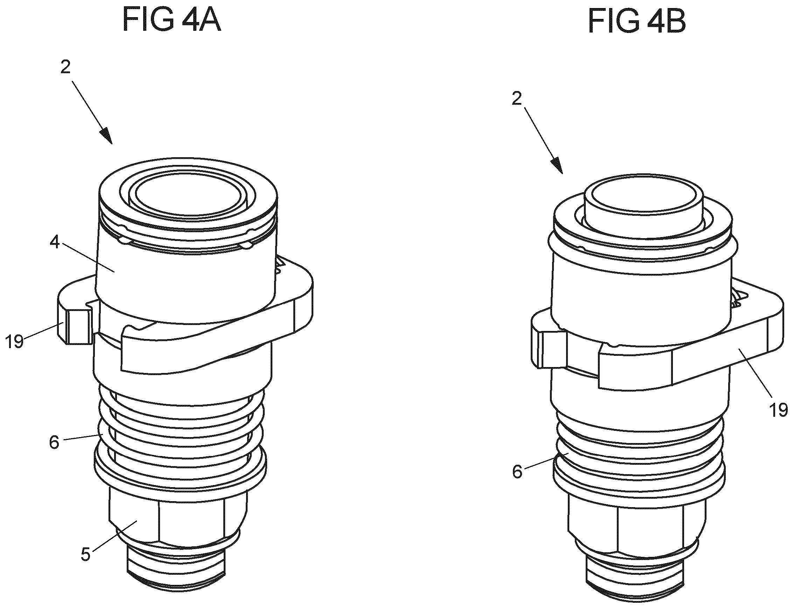

Die Figuren 4A und 4B zeigen ein weiteres Ausführungsbeispiel eines Adapters 2, wobei der Adapter 2 in der Figur 4A in seiner entkoppelten Position (Freigabeposition) dargestellt ist, während er in der Figur 4B in seiner gekoppelten Position (Rastposition) dargestellt ist, in der ein Hohlkörperinstrument 3 an dem Adapter 2 festgelegt ist.

Wie auch die in den Figuren 1A bis 1F und 2A bis 2F dargestellten Ausführungsbeispiele weist der Adapter 2 ein Außenteil 4 und ein Innenteil 5 auf, die lateral relativ zueinander beweglich sind. Ferner ist ein Verriegelungsclip 19 vorgesehen, der in seiner in der Figur 4A dargestellten Entriegelungsposition eine relative Bewegung zwischen dem Außenteil 4 und dem Innenteil 5 des Adapters 2 gestattet, während eine solche Bewegung in der Verriegelungsposition des Verriegelungsclips 19, die in der Figur 4B dargestellt ist, nicht mehr möglich ist. Dafür sorgt die spezifische Konturierung des Verriegelungsclips 19.

Durch eine erste Feder 6 ist der Adapter 2 in seine entkoppelte Position, in der eine Rasteinrichtung des Adapters 2 in ihrer Freigabeposition vorliegt, vorgespannt. Dies ist in der Figur 4A zu sehen. Wird das Außenteil 4 entgegen der von der ersten Feder 6 aufgebrachten Federkraft relativ zum Innenteil 5 heruntergedrückt, wird der Adapter 2 in seine gekoppelte Position überführt, in der die Rasteinrichtung des Adapters 2 in ihrer Rastposition vorliegt.

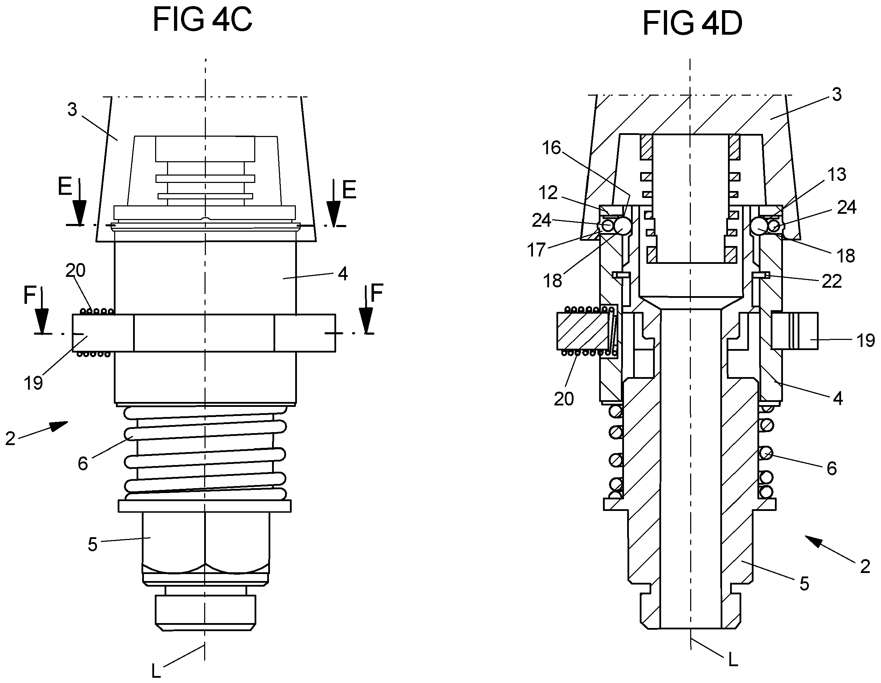

Die Figur 4C zeigt den Adapter 2 der Figur 4A in seiner entkoppelten Position in einer Seitenansicht. Dabei ist zu sehen, dass die zweite Feder 20, die den Verriegelungsclip 19 in seine Verriegelungsposition vorspannt, in der entkoppelten Position des Adapters 2 komprimiert ist. Das heißt, es wurde eine Kraft entgegen der von der zweiten Feder 20 ausgeübten Federkraft aufgebracht, um die Feder 20 zu komprimieren und den Verriegelungsclip 19 von seiner Verriegelungsposition in seine Entriegelungsposition zu überführen, in der eine Lateralbewegung zwischen dem Außenteil 4 und dem Innenteil 5 des Adapters 2 entlang der Längserstreckungsachse L möglich ist. In der Darstellung der Figur 4C ist zudem ein auf den Adapter 2 aufgesetztes Hohlkörperinstrument 3 ausschnittsweise zu sehen.

Die Figur 4D zeigt eine Längsschnittansicht durch den Adapter 2 der Figur 4C. In dieser Längsschnittansicht wird die Rasteinrichtung des Adapters 2 sichtbar, die - ähnlich wie die Rasteinrichtung der in den Figuren 1A bis 1F und 2A bis 2F dargestellten Adapter einen Durchbruch 13 aufweist. In dem Durchbruch 13 ist allerdings nicht nur eine Kugel 18 angeordnet. Vielmehr wird die Kugel 18 von einem Sprengring 24 umgeben, der als Bewegungsbegrenzungseinrichtung dient. Mithin ist es in dieser Ausgestaltung nicht erforderlich, dass die zweite Öffnung 17, die auf der radial äußeren Seite des Durchbruchs 13 ausgebildet ist, einen Durchmesser hat, der kleiner ist als der Durchmesser der Kugel 18. Vielmehr sorgt der Sprengring 24 dafür, dass die Kugel 18 den Durchbruch 13 nicht nach außen durch die zweite Öffnung 17 hindurch verlassen kann.

Demgegenüber ist es für die Kugel 18 nach wie vor möglich, den Durchbruch 13 durch die erste Öffnung 16 zu einer auf der Außenseite des Innenteils 5 ausgebildeten Aussparung zumindest teilweise zu verlassen. Insgesamt ist der Raum, der der Kugel 18 und dem Sprengring 24 zur Verfügung steht, jedoch größer als der Raum, den diese beiden Elemente benötigen. Mithin ist eine freie Beweglichkeit der Kugel 18 zwischen der Außenseite des Innenteils 5 und dem Sprengring 24 in dem Durchbruch 13 möglich. Mithin wird auf das Hohlkörperinstrument 3 keine Rastkraft ausgeübt, weshalb das Hohlkörperinstrument 3 problemlos vom Adapter 2 entfernt werden kann oder auf den Adapter 2 aufgesteckt werden kann.

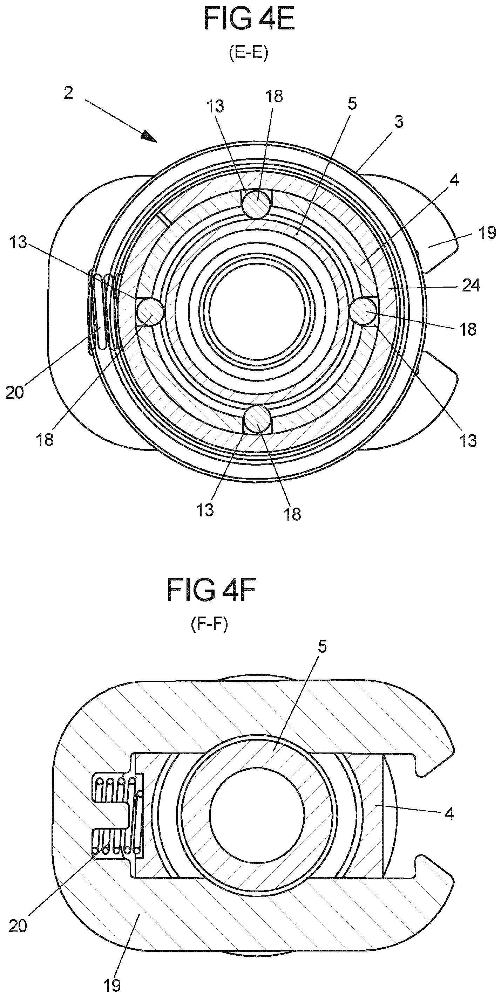

Die Figur 4E zeigt eine Querschnittansicht durch den Adapter 2 der Figur 4C entlang der dort mit E gekennzeichneten Linie. Somit zeigt die Figur 4E den Adapter 2 auf der Ebene der Rasteinrichtung. Wie aus der Figur 4E zu sehen ist, weist die Rasteinrichtung vier gleichmäßig entlang des Umfangs des Außenteils 4 angeordnete Durchbrüche 13 auf, in denen jeweils eine Kugel 18 angeordnet ist. Während die Kugeln 18 jeweils durch die innere Öffnung 16 hindurch aus der Außenteilwandung 12 in eine Ausnehmung der Außenseite des Innenteils 5 hineinragen können, ist ihre Bewegung radial nach außen durch den Sprengring 24 limitiert. Auf diese Weise wird verhindert, dass die Kugeln 18 aus dem Adapter 2 herausfallen können.

Die Figur 4F zeigt eine Querschnittsansicht durch den Adapter 2 entlang der mit F gekennzeichneten Linie in der Figur 4C. Diese Querschnittsdarstellung zeigt den Adapter 2 somit auf der Höhe des Verriegelungsclips 19. Wie aus der Figur 4F zu sehen ist, befindet sich der Verriegelungsclip 19 in seiner Entriegelungsposition, in der eine Relativbewegung zwischen dem Außenteil 4 und dem Innenteil 5 des Adapters 2 möglich ist, da der Verriegelungsclip 19 eine entsprechende kreisrunde Ausnehmung in seiner Mitte aufweist.

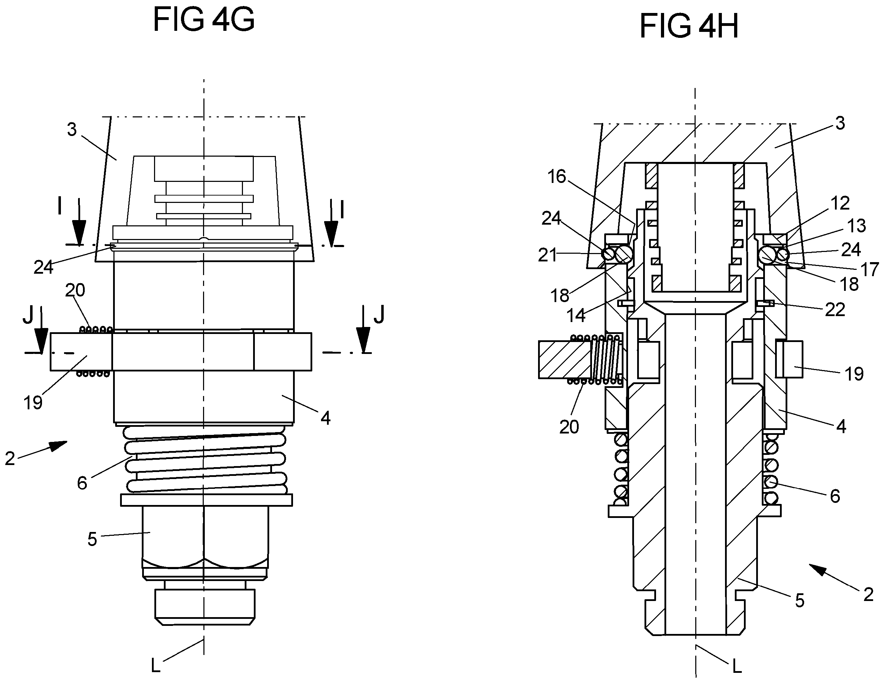

Die Figur 4G zeigt eine Seitenansicht des Adapters 2 der Figur 4B, also des Adapters 2 in seiner gekoppelten Position. In dieser gekoppelten Position ist das Hohlkörperinstrument 3 an dem Adapter 2 festgelegt. Mithin befindet sich die Rasteinrichtung des Adapters 2 in ihrer Rastposition. Die Rasteinrichtung wurde von der Freigabeposition, die in den Figuren 4A, 4B und 4C dargestellt ist, in die Rastposition der Figur 4G überführt, indem das Außenteil 4 und das Innenteil 5 des Adapters 2 entlang der Längserstreckungsachse L relativ zueinander bewegt wurden. Durch diese Bewegung wird die erste Feder 6 komprimiert.

Die Auswirkungen der relativen Lateralbewegung des Außenteils 4 zum Innenteil 5 auf die Rasteinrichtung des Adapters 2 sind in der Längsschnittansicht der Figur 4H zu sehen. So wird durch die relative Bewegung des Außenteils 4 zum Innenteil 5 der Hohlraum zwischen der Innenseite 14 der Außenteilwandung 12 und der Außenseite des Innenteils 5 verkleinert. Dadurch wird die Kugel 18 radial nach außen gedrückt. Der Sprengring 24 verhindert als Bewegungsbegrenzungseinrichtung eine beliebig weite Bewegung der Kugel 18. Er wird durch die Kugel 18 und den durch sie aufgebrachten Druck aber ebenfalls nach außen gedrückt, und zwar in eine Nut 21 des Hohlkörperinstruments 3. Dadurch erfolgt eine formschlüssige Verbindung zwischen dem Hohlkörperinstrument 3 und dem Adapter 2, sodass das Hohlkörperinstrument 3 sicher am Adapter 2 festgelegt ist.

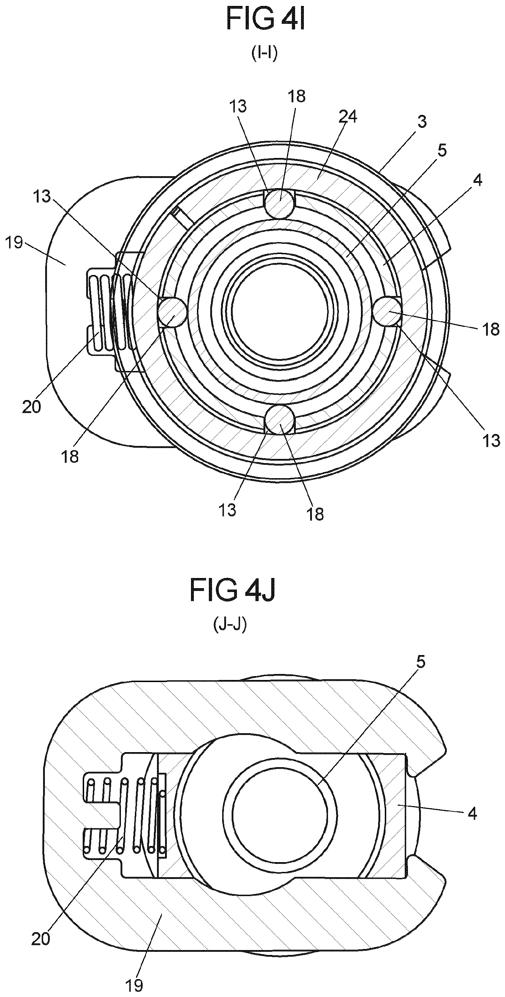

Die Figur 4I zeigt eine Querschnittsdarstellung entlang der mit I gekennzeichneten Linie in der Figur 4G, also auf Höhe der Rasteinrichtung des Adapters 2. Dabei befindet sich die Rasteinrichtung in ihrer Rastposition. Gerade im direkten Vergleich mit der Figur 4E, die die Rasteinrichtung in ihrer Freigabeposition zeigt, wird ersichtlich, dass die Kugeln 18 durch das Innenteil 5 des Adapters 2 radial weiter nach außen gedrückt sind und damit den Sprengring 24 aufweiten. Der Sprengring 24 übt somit eine Rastkraft auf das Hohlkörperinstrument 3 aus und greift in eine in dem Hohlkörperinstrument 3 ausgebildete Nut 21 ein, sodass sich auch eine formschlüssige Verbindung zwischen dem Adapter 2 und dem Hohlkörperinstrument 3 ergibt.

Die Figur 4J zeigt eine Querschnittsansicht entlang der mit J gekennzeichneten Linie in der Figur 4 G, also auf Höhe des Verriegelungsclips 19. In dieser Darstellung ist zu sehen, dass der Verriegelungsclip 19 in der gekoppelten Position zwischen dem Hohlkörperinstrument 3 und dem Adapter 2 in seiner Verriegelungsposition vorliegt, in der eine Relativbewegung zwischen dem Innenteil 5 und dem Außenteil 4 des Adapters 2 nicht mehr möglich ist, da die Konturierung des Verbindungsclips 19 eine solche Relativbewegung verhindert. Die zweite Feder 20 befindet sich dabei in ihrem entspannten Zustand. Mithin spannt sie den Verbindungsclip 19 in diese Verriegelungsposition vor. Wenn der Adapter 2 das Hohlkörperinstrument 3 wieder freigeben soll, muss zuerst eine der von der zweiten Feder 20 aufgebrachten Federkraft entgegengesetzte Kraft ausgeübt werden, um den Verriegelungsclip 19 wieder in seine Entriegelungsposition zu überführen, in der er eine relative Lateralbewegung des Innenteils 5 gegenüber dem Außenteil 4 des Adapters 2 gestattet. Dann wird die Rasteinrichtung des Adapters 2 von ihrer Rastposition in ihre Freigabeposition überführt, sodass des Hohlkörperinstrument 3 vom Adapter 2 entfernt werden kann.

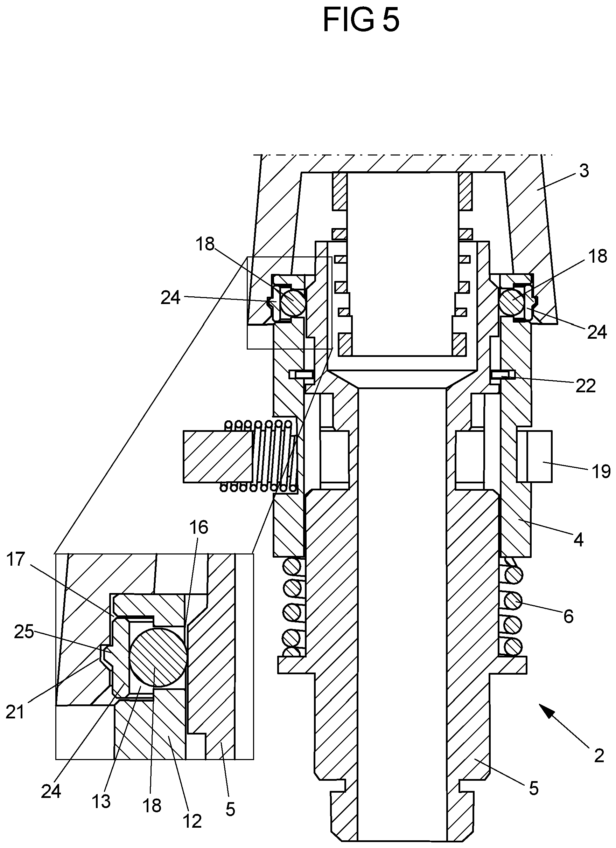

Die Figur 5 zeigt eine Längsschnittansicht durch ein weiteres Ausführungsbeispiel eines Adapters 2, das sehr ähnlich zu dem in der Figur 4H dargestellten Ausführungsbeispiel ausgestaltet ist. Insofern wird auf die obigen Erläuterungen verwiesen. Dabei wird der Adapter 2 in seiner gekoppelten Position gezeigt, also in einer Position, in der die Rasteinrichtung des Adapters 2 in ihrer Rastposition vorliegt.

Im Gegensatz zu dem in der Figur 4H dargestellten Ausführungsbeispiel weist der Adapter 2 der Figur 5 keinen Sprengring mit kreisförmigem Querschnitt, sondern einen Sprengring 24 mit einer speziell ausgestalteten Kontur auf. Dies ist in der als Einsatz in der Figur 5 wiedergegebenen vergrößerten Darstellung der Rasteinrichtung sichtbar. So weist der Sprengring 24 eine zur Kugel 18 hin orientierte plane Innenseite auf, während er an seiner Außenseite eine Nase 25 aufweist, die derart geformt ist, dass sie in die Nut 21 des Hohlkörperinstruments 3 eingreifen kann. Grundsätzlich kann der Sprengring 24 jede beliebige Form annehmen, die geeignet ist, um eine sichere Verbindung zwischen dem Adapter 2 und dem Hohlkörperinstrument 3 zu realisieren. Die in der Figur 5 dargestellte Ausgestaltung des Sprengrings 24 ist somit nur exemplarisch zu verstehen.

Ebenso kann die Konturierung der Außenseite des Innenteils 5 des Adapters 2 beliebig ausgestaltet werden, um die Bewegung der Kugel 18 von der Freigabeposition zur Rastposition der Rasteinrichtung entsprechend den jeweiligen Anforderungen zu beeinflussen. So wird die Kugel 18 beim Adapter 2 der Figur 5 in der Rastposition stärker radial nach außen gedrückt, als dies bei dem in der Figur 4H dargestellten Adapter 2 der Fall ist. Gleichzeitig weist der Sprengring 24 des Adapters 2 der Figur 5 einen etwas geringeren Radialdurchmesser als der Sprengring 24 des Adapters 2 der Figur 4H auf. Somit ist eine individuelle Anpassbarkeit des Adapters 2 an die jeweils zu kontaktierenden Hohlkörperinstrumente 3 möglich, was zahlreiche Anwendungsmöglichkeiten für den Adapter 2 eröffnet.

## Patentansprüche

1. Adapter (2) zum Verbinden eines Hohlkörperobjekts (3) mit einem Gerät zur Innendurchspülung von Hohlkörperobjekten,
wobei der Adapter (2) ein Innenteil (5) und ein das Innenteil (5) zumindest abschnittsweise umgebendes Außenteil (4) aufweist,
wobei das Innenteil (5) und das Außenteil (4) relativ zueinander beweglich sind und sich entlang einer Längserstreckungsachse (L) erstrecken,
wobei das Außenteil (4) eine Außenteilwandung (12) mit mindestens einer Rasteinrichtung aufweist,
**dadurch gekennzeichnet,**
**dass** die Rasteinrichtung einen Durchbruch (13) in der Außenteilwandung (12), einen in dem Durchbruch (13) beweglich gelagerten Körper (18) und eine Bewegungsbegrenzungseinrichtung (17, 24) umfasst,
wobei der Durchbruch eine erste Öffnung (16) in einer zum Innenteil (5) orientierten Innenseite (14) der Außenteilwandung (12) und eine zweite Öffnung (17) in einer Außenseite (15) der Außenteilwandung (12) aufweist, wobei eine Radialrichtung von der ersten Öffnung (16) zur zweiten Öffnung (17) verläuft,
wobei die Bewegungsbegrenzungseinrichtung (17, 24) eine in der Radialrichtung zur zweiten Öffnung (17) verlaufende Bewegung des Körpers (18) begrenzt und verhindert, dass der Körper (18) vollständig durch die zweite Öffnung (17) nach außen durchtreten kann,
wobei die erste Öffnung (16) einen Durchmesser aufweist, der größer als ein Querschnittsdurchmesser des Körpers (18) ist,
wobei die Rasteinrichtung durch eine mittels einer Relativbewegung zwischen dem Außenteil (4) und dem Innenteil (5) erfolgende Bewegung zwischen einer Freigabeposition und einer Rastposition verstellbar ist,
wobei der Körper (18) im Durchbruch (13) in der Rastposition radial weiter außen angeordnet ist als in der Freigabeposition,
wobei eine Außenseite der Rasteinrichtung in der Freigabeposition nicht über eine Außenumhüllende der Außenteilwandung (12) hinausragt, in der Rastposition jedoch über die Außenumhüllende der Außenteilwandung (12) hinausragt.

2. Adapter nach Anspruch 1, **dadurch gekennzeichnet, dass** die Bewegungsbegrenzungseinrichtung durch einen Sprengring (24) ausgebildet ist, der in dem Durchbruch (13) in der Radialrichtung außen um den Körper (18) herum angeordnet ist.

3. Adapter nach Anspruch 2, **dadurch gekennzeichnet, dass** die Außenteilwandung (12) im Bereich der Rasteinrichtung eine Wandstärke aufweist, die kleiner ist als eine Erstreckung des Körpers (18) und des Sprengrings (24) in der Radialrichtung.

4. Adapter nach Anspruch 1, **dadurch gekennzeichnet, dass** die Bewegungsbegrenzungseinrichtung durch einen Durchmesser der zweiten Öffnung (17) ausgebildet ist, der kleiner als der Querschnittsdurchmesser des Körpers (18) ist.

5. Adapter nach Anspruch 4, **dadurch gekennzeichnet, dass** die Außenteilwandung (12) im Bereich der Rasteinrichtung eine Wandstärke aufweist, die kleiner ist als eine Erstreckung des Körpers (18) in der Radialrichtung.

6. Adapter nach einem der vorherigen Ansprüche, **dadurch gekennzeichnet, dass** der Adapter (2) ein Verriegelungselement (19) aufweist, das relativ zum Außenteil (4) und zum Innenteil (5) zwischen einer Entriegelungsposition und einer Verriegelungsposition beweglich ist, wobei das Verriegelungselement (19) in der Entriegelungsposition die Relativbewegung zwischen dem Außenteil (4) und dem Innenteil (5) gestattet und in der Verriegelungsposition diese Relativbewegung verhindert.

7. Adapter nach Anspruch 6, **dadurch gekennzeichnet, dass** das Verriegelungselement (19) quer zur Längserstreckungsachse (L) zwischen der Entriegelungsposition und der Verriegelungsposition beweglich ist.

8. Adapter nach Anspruch 6 oder 7, **dadurch gekennzeichnet, dass** das Verriegelungselement (19) mit einem ersten Vorspannelement (20) in die Verriegelungsposition vorgespannt ist.

9. Adapter nach einem der vorherigen Ansprüche, **dadurch gekennzeichnet, dass** das Innenteil (5) eine zum Außenteil (4) orientierte Querschnittsverjüngung aufweist, die in der Freigabeposition mit dem Durchbruch (13) fluchtet, so dass der Körper (13) in die Querschnittsverjüngung hineinragen kann.

10. Adapter nach Anspruch 9, **dadurch gekennzeichnet, dass** die Querschnittsverjüngung in der Rastposition nicht mit dem Durchbruch (13) fluchtet.

11. Adapter nach einem der vorherigen Ansprüche, **dadurch gekennzeichnet, dass** der Körper (18) in der Freigabeposition vorspannungsfrei in dem Durchbruch (13) gelagert ist.

12. Adapter nach einem der vorherigen Ansprüche, **dadurch gekennzeichnet, dass** genau ein Körper (18) in dem Durchbruch (13) gelagert ist.

13. Adapter nach einem der vorherigen Ansprüche, **dadurch gekennzeichnet, dass** der Adapter (2) mindestens zwei Rasteinrichtungen aufweist, die insbesondere gleichmäßig entlang einer Umfangsrichtung des Außenteils (4) im Außenteil (4) angeordnet sind.

14. Adapteranordnung (1), umfassend einen Adapter (2) nach einem der vorherigen Ansprüche und ein auf einer Außenseite (15) des Außenteils (4) angeordnetes Hohlkörperobjekt (3), das in der Freigabeposition der Rasteinrichtung lösbar mit dem Adapter (2) verbunden ist und in der Rastposition der Rasteinrichtung i) formschlüssig oder ii) form- und kraftschlüssig oder iii) kraftschlüssig mit dem Adapter (2) verbunden ist.

15. Verfahren zum Verbinden eines Hohlkörperobjekts (3) mit einem Gerät zur Innendurchspülung von Hohlkörperobjekten und Lösen des Hohlkörperobjekts von dem Gerät, **gekennzeichnet durch** die folgenden Schritte:
a) Aufstecken eines Hohlkörperobjekts (3) auf eine Außenseite (15) eines Adapters (2) nach einem der Ansprüche 1 bis 13;
b) relative Bewegung des Außenteils (4) und des Innenteils (5) des Adapters (2) zueinander, um die Rasteinrichtung von der Freigabeposition in die Rastposition zu überführen;
c) relative Bewegung des Außenteils (4) und des Innenteils (5) des Adapters (2) zueinander, um die Rasteinrichtung von der Rastposition in die Freigabeposition zu überführen; und
d) Entfernen des Hohlkörperobjekts (3) von der Außenseite (15) des Adapters (2).

## Claims

1. An adapter (2) for connecting a hollow-body object (3) to a device for washing through the inside of hollow-body objects,
wherein the adapter (2) comprises an inner part (5) and an outer part (4) at least sectionally surrounding the inner part (5),
wherein the inner part (5) and the outer part (4) are movable relative to each other and extend along a longitudinally extending axis (L),
wherein the outer part (4) comprises an outer part wall (12) with at least one latching device,
**characterized in**
**that** the latching device comprises a break-through (13) in the outer part wall (12), a body (18) movably mounted in the break-through (13) and a movement limiting device (17, 24),
wherein the break-through comprises a first opening (16) in an inside (14) of the outer part wall (12) oriented towards the inner part (5) and a second opening (17) in an outside (15) of the outer part wall (12), wherein a radial direction extends from the first opening (16) to the second opening (17),
wherein the movement limiting device (17, 24) limits a movement of the body (18) extending in the radial direction to the second opening (17) and prevents that the body (18) can completely pass through the second opening (17) to the outside,
wherein the first opening (16) has a diameter which is greater than a cross-sectional diameter of the body (18),
wherein the latching device can be adjusted between a release position and a latching position by a movement effected by means of a relative movement between the outer part (4) and the inner part (5),
wherein in the latching position the body (18) is arranged in the breakthrough (13) radially further to the outside than in the release position,
wherein in the release position an outside of the latching device does not protrude beyond an outer circumference of the outer part wall (12), but in the latching position extends beyond the outer envelope of the outer part wall (12).

2. The adapter according to claim 1, **characterized in that** the movement limiting device is formed by a snap ring (24) which is arranged in the break-through (13) in the radial direction around the outside of the body (18).

3. The adapter according to claim 2, **characterized in that** in the region of the latching device the outer part wall (12) has a wall thickness which is smaller than an extension of the body (18) and of the snap ring (24) in the radial direction.

4. The adapter according to claim 1, **characterized in that** the movement limiting device is formed by a diameter of the second opening (17) which is smaller than the cross-sectional diameter of the body (18).

5. The adapter according to claim 4, **characterized in that** in the region of the latching device the outer part wall (12) has a wall thickness which is smaller than an extension of the body (18) in the radial direction.

6. The adapter according to any of the preceding claims, **characterized in that** the adapter (2) comprises a locking element (19) which is movable relative to the outer part (4) and to the inner part (5) between an unlocking position and a locking position, wherein in the unlocking position the locking element (19) permits the relative movement between the outer part (4) and the inner part (5) and in the locking position prevents this relative movement.

7. The adapter according to claim 6, **characterized in that** the locking element (19) is movable transversely to the longitudinally extending axis (L) between the unlocking position and the locking position.

8. The adapter according to claim 6 or 7, **characterized in that** the locking element (19) is pretensioned into the locking position by means of a first pretensioning element (20).

9. The adapter according to any of the preceding claims, **characterized in that** the inner part (5) has a cross-sectional taper oriented towards the outer part (4), which in the release position is aligned with the break-through (13) so that the body (18) can protrude into the cross-sectional taper.

10. The adapter according to claim 9, **characterized in that** in the latching position the cross-sectional taper is not aligned with the break-through (13).

11. The adapter according to any of the preceding claims, **characterized in that** in the release position the body (18) is mounted in the break-through (13) free from pretension.

12. The adapter according to any of the preceding claims, **characterized in that** exactly one body (18) is mounted in the break-through (13).

13. The adapter according to any of the preceding claims, **characterized in that** the adapter (2) comprises at least two latching devices which in particular are uniformly arranged in the outer part (4) along a circumferential direction of the outer part (4).

14. An adapter assembly (1), comprising an adapter (2) according to any of the preceding claims and a hollow-body object (3) arranged on an outside (15) of the outer part (4), which in the release position of the latching device is releasably connected to the adapter (2) and in the latching position of the latching device is i) positively or ii) positively and non-positively or iii) non-positively connected to the adapter (2).

15. A method for connecting a hollow-body object (3) to a device for washing through the inside of hollow-body objects and releasing the hollow-body object from the device, **characterized by** the following steps:
a) pushing a hollow-body object (3) onto an outside (15) of an adapter (2) according to any of claims 1 to 13;
b) moving the outer part (4) and the inner part (5) of the adapter (2) relative to each other, in order to transfer the latching device from the release position into the latching position;
c) moving the outer part (4) and the inner part (5) of the adapter (2) relative to each other, in order to transfer the latching device from the latching position into the release position; and
d) removing the hollow-body object (3) from the outside (15) of the adapter (2).

## Revendications

1. Adaptateur (2) pour relier un objet à corps creux (3) à un appareil pour le rinçage intérieur d'objets à corps creux,
l'adaptateur (2) présentant une partie intérieure (5) et une partie extérieure (4) entourant la partie intérieure (5) au moins par sections,
la partie intérieure (5) et la partie extérieure (4) étant mobiles l'une par rapport à l'autre et s'étendant le long d'un axe d'extension longitudinal (L),
la partie extérieure (4) présentant une paroi de partie extérieure (12) avec au moins un dispositif d'encliquetage,
**caractérisé en ce**
**que** le dispositif d'encliquetage comprend un passage (13) dans la paroi de partie extérieure (12), un corps (18) monté mobile dans le passage (13) et un dispositif de limitation de mouvement (17, 24),
le passage présentant une première ouverture (16) dans une face intérieure (14) de la paroi de partie extérieure (12) orientée vers la partie intérieure (5) et une deuxième ouverture (17) dans une face extérieure (15) de la paroi de partie extérieure (12), une direction radiale s'étendant de la première ouverture (16) à la deuxième ouverture (17),
le dispositif de limitation de mouvement (17, 24) limitant un mouvement du corps (18) s'étendant dans la direction radiale vers la deuxième ouverture (17) et empêche le corps (18) de passer complètement à travers la deuxième ouverture (17) vers l'extérieur,
la première ouverture (16) présentant un diamètre qui est supérieur à un diamètre de section transversale du corps (18),
le dispositif d'encliquetage étant réglable entre une position de libération et une position d'encliquetage par un mouvement effectué au moyen d'un mouvement relatif entre la partie extérieure (4) et la partie intérieure (5),
le corps (18) étant disposé radialement plus à l'extérieur dans le passage (13) dans la position d'encliquetage que dans la position de libération,
un côté extérieur du dispositif d'encliquetage ne faisant pas saillie au-delà d'une enveloppe extérieure de la paroi de partie extérieure (12) dans la position de libération, mais fait saillie au-delà de l'enveloppe extérieure de la paroi de partie extérieure (12) dans la position d'encliquetage.

2. Adaptateur selon la revendication 1, **caractérisé en ce que** le dispositif de limitation de mouvement est formé par un jonc d'arrêt (24) qui est agencé dans le passage (13) autour de l'extérieur du corps (18) dans la direction radiale.

3. Adaptateur selon la revendication 2, **caractérisé en ce que** la paroi de partie extérieure (12) présente, dans la zone du dispositif d'encliquetage, une épaisseur de paroi qui est inférieure à une extension du corps (18) et du jonc d'arrêt (24) dans la direction radiale.

4. Adaptateur selon la revendication 1, **caractérisé en ce que** le dispositif de limitation de mouvement est formé par un diamètre de la deuxième ouverture (17) qui est inférieur au diamètre de la section transversale du corps (18).

5. Adaptateur selon la revendication 4, **caractérisé en ce que** la paroi de partie extérieure (12) présente, dans la zone du dispositif d'encliquetage, une épaisseur de paroi qui est inférieure à une extension du corps (18) dans la direction radiale.

6. Adaptateur selon l'une quelconque des revendications précédentes, **caractérisé en ce que** l'adaptateur (2) comprend un élément de verrouillage (19) qui est mobile par rapport à la partie extérieure (4) et à la partie intérieure (5) entre une position de déverrouillage et une position de verrouillage, l'élément de verrouillage (19) dans la position de déverrouillage permettant le mouvement relatif entre la partie extérieure (4) et la partie intérieure (5) et dans la position de verrouillage empêchant ce mouvement relatif.

7. Adaptateur selon la revendication 6, **caractérisé en ce que** l'élément de verrouillage (19) est mobile transversalement à l'axe d'extension longitudinal (L) entre la position de déverrouillage et la position de verrouillage.

8. Adaptateur selon la revendication 6 ou 7, **caractérisé en ce que** l'élément de verrouillage (19) est précontraint dans la position de verrouillage par un premier élément de précontrainte (20).

9. Adaptateur selon l'une quelconque des revendications précédentes, **caractérisé en ce que** la partie intérieure (5) présente un rétrécissement de section transversale orienté vers la partie extérieure (4) qui, en position de libération, est aligné avec le passage (13), de sorte que le corps (18) peut pénétrer dans le rétrécissement de section transversale.

10. Adaptateur selon la revendication 9, **caractérisé en ce que** le rétrécissement de section transversale n'est pas aligné avec le passage (13) dans la position d'encliquetage.

11. Adaptateur selon l'une quelconque des revendications précédentes, **caractérisé en ce que** le corps (18) est monté sans précontrainte dans le passage (13) dans la position de libération.

12. Adaptateur selon l'une quelconque des revendications précédentes, **caractérisé en ce que** exactement un corps (18) est monté dans le passage (13).

13. Adaptateur selon l'une quelconque des revendications précédentes, **caractérisé en ce que** l'adaptateur (2) comprend au moins deux dispositifs d'encliquetage qui sont agencés dans la partie extérieure (4), en particulier uniformément le long d'une direction circonférentielle de la partie extérieure (4).

14. Ensemble adaptateur (1), comprenant un adaptateur (2) selon l'une quelconque des revendications précédentes et un objet à corps creux (3) agencé sur un côté extérieur (15) de la partie extérieure (4), qui est relié de manière amovible à l'adaptateur (2) dans la position de libération du dispositif d'encliquetage et qui est relié à l'adaptateur (2) dans la position d'encliquetage du dispositif d'encliquetage i) par complémentarité de formes ou ii) par complémentarité de formes et de forces ou iii) par complémentarité de forces.

15. Procédé pour relier un objet à corps creux (3) à un appareil pour le rinçage intérieur d'objets à corps creux et pour détacher l'objet à corps creux de l'appareil, **caractérisé par** les étapes suivantes :
a) emboîtement d'un objet à corps creux (3) sur une face extérieure (15) d'un adaptateur (2) selon l'une quelconque des revendications 1 à 13 ;
b) un mouvement relatif de la partie extérieure (4) et de la partie intérieure (5) de l'adaptateur (2) l'une par rapport à l'autre, afin de faire passer le dispositif d'encliquetage de la position de libération à la position d'encliquetage ;
c) un mouvement relatif de la partie extérieure (4) et de la partie intérieure (5) de l'adaptateur (2) l'une par rapport à l'autre, afin de faire passer le dispositif d'encliquetage de la position d'encliquetage à la position de libération ; et
d) enlever l'objet à corps creux (3) du côté extérieur (15) de l'adaptateur (2).
